# EUROPEAN PATENT SPECIFICATION

(11) **EP 1 951 913 B1**
(45) Date of publication and mention of the grant of the patent: **10.09.2014**
(21) Application number: 06851884.4
(22) Date of filing: 20.09.2006
(51) Int. Cl.: C12Q 1/00, G01N 33/48, G01N 33/50

(54) **PROCESS TO DETECT BINDING EVENTS ON AN ELECTRODE MICROARRAY USING ENZYMES**
VERFAHREN ZUR ERKENNUNG VON BINDUNGSEREIGNISSEN AUF EINEM ELEKTRODEN-MIKROARRAY MITHILFE VON ENZYMEN
PROCEDE POUR DETECTER DES EVENEMENTS DE LIAISON SUR UN MICRORESEAU D'ELECTRODES A L'AIDE D'ENZYMES

(30) Priority: 21.09.2005 US 232479
(43) Date of publication of application: 06.08.2008
(73) Proprietor: Combimatrix Corporation, Mukilteo, WA 98275 (US)
(72) Inventor: DILL, Kilian, Monroe, WA 98272 (US); COOPER, John J., Seattle, WA 98115 (US); GHINDILIS, Andrei, Mukilteo, WA 98275 (US); ROTH, Kristian M., Bothell, WA 98012 (US)
(74) Representative: de Lang, Robbert-Jan
(86) International application number: PCT/US2006/036616
(87) International publication number: WO 2008/051196

(56) References cited:
- WO-A-00/54882
- WO-A-99/67628
- WO-A-03/019147
- US-A- 5 632 957
- US-A- 6 093 302
- US-A1- 2004 023 258
- US-A1- 2004 072 158
- LOWE C R: "Chemoselective biosensors" CURRENT OPINION IN CHEMICAL BIOLOGY, CURRENT BIOLOGY LTD, LONDON, GB, vol. 3, no. 1, 1 February 1999 (1999-02-01), pages 106-111, XP002971674 ISSN: 1367-5931
- CHENG J ET AL: "PREPARATION AND HYBRIDIZATION ANALYSIS OF DNA/RNA FROM E. COLI ON MICROFABRICATED BIOELECTRONIC CHIPS" NATURE BIOTECHNOLOGY, NATURE PUBLISHING GROUP, NEW YORK, NY, US, vol. 16, 1 June 1998 (1998-06-01), pages 541-546, XP000992691 ISSN: 1087-0156
- NABID ET AL.: 'A Novel Method for Synthesis of Water-Soluble Polypyrrole with Horseradish Peroxidase Enzyme' J. APPL. POLYMER SCI. vol. 94, 2004, pages 254 - 258, XP003023747
- FIACCABRINO G C ET AL: "Array of individually addressable microelectrodes", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 19, no. 1-3, 1 April 1994 (1994-04-01), pages 675-677, XP026570910, ISSN: 0925-4005, DOI: 10.1016/0925-4005(93)01126-O [retrieved on 1994-04-01]

## Description

### Technical Field of the Invention

This invention provides a process for electronic detection of binding events between probes attached to known locations on a microarray device having electrodes and targets in solution. Detection is accomplished using enzymatic moieties and substrate solutions that alter the electrical properties of electrodes having the enzymatic moieties.

### Background of the Invention

Within biotechnology research and discovery, microarrays have become important analytical research tools. In general, microarrays are miniaturized arrays of locations on a solid surface, which is usually planar. As part of the preparation of a microarray, the locations may have presynthesized molecules, including biomolecules, attached thereto or may have molecules synthesized *in situ* such as a DNA molecule synthesized one monomer at a time. The attachment locations are usually in a column and row format; however, other formats may be used. Most often, microarrays, and in particular, microarrays of oligonucleotides, are silicon-based and most often are a glass microscope slide. The major advantage of microarrays is the ability to conduct hundreds, if not thousands, of experiments simultaneously. Simultaneous experimentation increases the efficiency of exploring relationships between molecular structure and biological function, wherein slight variations in chemical structure can have profound biochemical effects. As the name suggests, the attachment points on microarrays are of a micrometer scale, which is generally 1-100µm.

Research using microarrays has focused mainly on deoxyribonucleic acid (DNA) and ribonucleic acid (RNA) related areas, which includes genomics, cellular gene expression, single nucleotide polymorphisms (SNP), genomic DNA detection and validation, functional genomics, and proteomics (Wilgenbus and Lichter, J. Mol. Med. 77:761, 1999; Ashfari et al., Cancer Res. 59:4759, 1999; Kurian et al., J. Pathol. 187:267, 1999; Hacia, Nature Genetics 21 suppl.:42, 1999; Hacia et al., Mol. Psychiatry 3:483, 1998; and Johnson, Curr. Biol. 26:R171, 1998.) Additionally, microarrays can be used for research related to peptides (two or more linked natural or synthetic amino acids), small molecules (such as pharmaceutical compounds), oligomers, and polymers. There are numerous methods for preparing a microarray of DNA related molecules, which includes native or cloned DNA and synthetic DNA. Synthetic, relatively short single-stranded DNA or RNA strands are commonly referred to as oligonucleotides.

Microarray preparation methods include the following: (1) spotting a solution on a prepared flat surface using spotting robots; (2) *in situ* synthesis by printing reagents via ink jet or other printing technology and using regular phosphoramidite chemistry; (3) *in situ* parallel synthesis using electrochemically-generated acid for deprotection and using regular phosphoramidite chemistry; (4) maskless photo-generated acid (PGA) controlled *in situ* synthesis and using regular phosphoramidite chemistry; (5) mask-directed *in situ* parallel synthesis using photo-cleavage of photolabile protecting groups (PLPG); (6) maskless *in situ* parallel synthesis using PLPG and digital photolithography; and (7) electric field attraction/repulsion for depositing oligonucleotides. A review of oligonucleotide microarray synthesis is provided by: Gao et al., Biopolymers 73:579, 2004.

WO03019147 discloses a method for detecting analytes, comprising: contacting a sample with an electrode microarray having immobilized antibodies; contacting the microarray with antibodies labeled with oxidoreductases; adding oxidoreductase substrates, and measuring the voltage or current generated by the oxidation or reduction of the substrate. The method uses a potentiostat and therefore measures the current flowing from the working electrode of the microarray to the reference electrode of the potentiostat itself.

Photolithographic techniques for *in situ* oligonucleotide synthesis are disclosed in Fodor et al. U.S. Patent No 5,445,934 and the additional patents claiming priority thereto. Electric field attraction/repulsion microarrays are disclosed in Hollis et al. U.S. Patent No. 5,653,939 and Heller et al. U.S. Patent No. 5,929,208. An electrode microarray for *in situ* oligonucleotide synthesis using electrochemical deblocking is disclosed in Montgomery, U.S. Patent Nos. 6,093,302, 6,280,595, and 6,444,111 (Montgomery I, II, and III respectively).

The electrochemical synthesis microarray disclosed in Montgomery I, II, and III is based upon a semiconductor chip having a plurality of microelectrodes in a column and row format. This chip design uses Complementary Metal Oxide Semiconductor (CMOS) technology to create high-density arrays of microelectrodes with parallel addressing for selecting and controlling individual microelectrodes within the microarray. In order to provide appropriate reactive groups at each electrode, the microarray is coated with a porous reaction matrix material (layer.) The thickness and porosity of the matrix are controlled. Biomolecules, as well as other molecules, can be synthesized at locations on any of the electrodes on the porous matrix.

During synthesis at a location, the electrode is "turned on" by applying a voltage or current that generates electrochemical reagents (particularly acidic protons) that alter the pH in a small, defined "virtual flask" region or volume adjacent to the electrode and within the porous matrix. The electrochemically-generated reagents remove protective groups on the molecule being synthesized to allow continued synthesis of a DNA or other oligomeric or polymeric material. The pH decreases only in the vicinity of the electrode because the ability of the acidic reagent to travel away from an electrode is limited by natural diffusion and by buffering.
The molecules synthesized or attached to a microarray are commonly referred to as probes. Each location on a microarray may have a different probe. During an experiment using a microarray, a target molecule in solution is allowed to interact with the microarray. If the target has sufficient affinity under the experimental conditions, it will bind to a specific probe type or possibly to more than one type of probe. Accurate detection of the target to probe binding event is required to capture data on the interaction of any target in solution with the probes on a microarray.

For microarrays, a photon-based detection system is generally used to detect a binding event. Most commonly, microarray detection processes use fluorescent tags on the targets for transduction of the binding event; the amount of fluorescence is a measure of the amount of binding. Alternatively, visible dyes or luminescent tags may be used. For example, for DNA hybridization, the tag is attached to target DNA sequences to detect hybridization to a probe oligonucleotide attached to a microarray. Depending upon the intensity of the signal from the tag, such microarrays may have to be read through laser confocal microscope-based system for microarrays configured in a monolayer (such as those microarrays made through high density spotting or photolithography techniques) or by a video-type camera (such as a CCD camera) for those microarrays having a three-dimensional matrix for each spot in high density formats.

An alternative to fluorescence has been optical detection of probe-target binding. In a "scanometric" assay, targets are labeled with catalytic gold particles. After binding with the probe, a silver salt is added to the solution and metallic silver is deposited where the particles are bound. Detection is similar to optical photographic film development and is recorded using either a digital scanner or photographic techniques. This technique does alleviate some of the technical demands of fluorescent detection but it is unclear how sensitive scanometric techniques will be at spot sizes relegated by current state of the art microarrays.

Generally, photon-based readers are expensive, relatively large and cumbersome, rely on sophisticated numerical algorithms, and must be accurately calibrated before use. Thus, use of such readers is generally limited to a laboratory setting. In each instance of "reading" the signal from a microarray, there is often stray light or other noise signals that cause false or inaccurate readings. Moreover, distinguishing between shades of gray or barely perceptible signals as true positives or false positives is difficult. Finally, there may be quenching of the fluorescent signal and auto absorption of the signal by other labels within close proximity to the bound target. The additional complexity associated with using a photon-based reader imparts added variability. Therefore, there is a need in the art for improvements to the detection process for analyzing binding events on microarrays. The present invention was made to address this need to improve detection of binding events and to provide a detection system that can generate a more objective "yes" or "no" answer for each location in high-density microarray system.

### Brief Summary of the Invention

The present invention provides a process to detect binding events on an electrode microarray using enzymes consisting of:
(a) providing a microarray having a plurality of electrodes, each electronically addressable, and having at least two different capture complexes attached at sites corresponding to the electrodes, wherein a first capture complex type has affinity for a first analyte type and second and subsequent capture complex types have affinity for subsequent analyte types, wherein the electrodes are electronically addressable, and wherein an addressable electrode is one where the electrode can be electronically controlled to create a current or voltage at the electrode;
(b) administering a plurality of analytes of at least one type to the capture complexes to form bound complexes of types corresponding to each type of the analytes captured by the capture complexes;
(c) attaching enzymes to the bound complexes to form reporter complexes, wherein the reporter complexes are of a type corresponding to each type of the analytes captured by the capture complexes;
(d) sequentially contacting a plurality of substrate solutions to the microarray and measuring for the presence or absence of an enzymatic product at the sites using an electrical signal, wherein each substrate solution corresponds to the reporter complex type, wherein the presence of the electrical signal is a measure of the binding event, and
wherein a low initial potential slightly positive to ground, preferably approximately 10 millivolt absolute, is applied to the electrodes while maintaining an open circuit to the electrodes; and wherein an electrical response of each electrode is measured by sequentially setting each electrode to ground, measuring the current flow from each electrode towards the remaining part of the electrode microarray used as a counter electrode, and returning each electrode to the initial potential at open circuit.

Preferably, the capture complexes are comprised of a plurality of probe oligonucleotides, the analytes are comprised of a plurality of target oligonucleotides, the bound complexes are formed by hybridization of the target oligonucleotides to the probe oligonucleotides, and the enzymes are bound to the target oligonucleotides by an attaching method, wherein the attaching method comprises attaching the enzymes through molecular groups selected from the group consisting of (a) an antibody, anti-antibody, and anti-idiotype antibody combination, (b) a biotin and streptavidin or avidin combination, and (c) an oligonucleotide and complementary oligonucleotide combination, and combinations thereof. Preferably, the probe oligonucleotides are synthesized by *in situ* electrochemical synthesis.

Preferably, the capture complexes are comprised of probe oligonucleotides hybridized to target oligonucleotides having antibody tags, the bound complexes are formed by capture of the analytes by the antibody tags, and the enzymes are bound to the analytes by an attaching method, wherein the attaching method comprises a reporter antibody attached to the analyte and the enzyme attached to the reporter antibody through molecular groups selected from the group consisting of (a) an antibody, anti-antibody, and anti-idiotype antibody combination, (b) a biotin and streptavidin or avidin combination, and (c) an oligonucleotide and complementary oligonucleotide combination, and combinations thereof.

Preferably, the probe oligonucleotides are synthesized by *in situ* electrochemical synthesis. Preferably, the capture complexes are made by a method selected from the group consisting of *in situ* electrochemical synthesis, spotting, ink-jet printing, electric field deposition, and *in situ* photolithography synthesis. Preferably, the capture complexes are comprised of molecules selected from the group consisting of oligonucleotides, polypeptides, antibodies, glycosylated polypeptides, polysaccharides, peptide nucleic acids, and mixed molecules having monomers from a plurality of the foregoing molecules.

Preferably, the analytes are comprised of molecules selected from the group consisting of antigens, haptens, viruses, bacteria, cells, proteins, polysugars, biological polymer molecules, lipids, glycoproteins (alpha-1-acid glycoprotein,) ricin, M13 phage, Bacillus globigii (BG) spores, fluorescein, rabbit IgG, goat IgG, DNA, RNA, single-stranded DNA, ribosomal RNA, mitochondrial DNA, cellular receptors, glycosylated membrane-bound proteins, non-glycosylated membrane-bound proteins, polypeptides, glycosylated polypeptides, antibodies, cellular antigenic determinants, organic molecules, metal ions, salt anions and cations, and organometallics, and combinations thereof.

Preferably, the enzymes are selected from the group consisting of horseradish peroxidase, laccase, beta-galactosidase, glucose oxidase, alkaline phosphatase, glucose-6-phosphate dehydrogenase, catalase, lactate oxidase, and peroxidase, and combinations thereof. Preferably, the substrate solutions are comprised of an aqueous solution having a buffer, a salt, and an enzymatic substrate solution, wherein the enzymatic substrate solution has substrates that are reactive with the enzyme. Preferably, the enzymatic product comprises a molecule that is electrochemically reducible or oxidizeable.

Preferably, the electrical signal comprises a difference response, wherein the difference response is a measure of the difference between a product response and a base response, wherein the product response is measured on the sites having enzyme and the base response is measured on sites not having enzyme, wherein the product response and the base response are selected from the group consisting of current, voltage, and resistance, wherein the difference response is a measure of binding of the analytes to the capture complexes.

Preferably, the enzyme product in step d) is a solid enzyme product that deposits on the sites having enzymes, and wherein a measurement solution and an electrical signal are used for the presence or absence of the solid enzyme product at the sites.

Preferably, the enzymes are selected from the group consisting of horseradish peroxidase, laccase, beta-galactosidase, glucose oxidase, alkaline phosphatase, and glucose dehydrogenase, and combinations thereof. Preferably, the substrate solutions are comprised of an aqueous solution having a buffer, a salt, and an enzymatic substrate solution, wherein the enzymatic substrate solutions has substrates that are reactive with the enzyme. Preferably, the enzyme is horseradish peroxidase and the substrate is hydrogen peroxide and a chemical selected from the group consisting of oxidizeable aromatics, ferrocene derivatives, and oxidizeable inorganic compounds, and combinations thereof.

Preferably, the solid enzyme product is non-reactive and the measurement solution is an aqueous solution having an electron mediator. Preferably, the electron mediator is a mixture of potassium ferrous cyanide and potassium ferric cyanide having a mixture proportion of approximately 10:90 to 90:10 and a concentration of approximately 0.1 to 1000 millimolar.

Alternatively, the solid enzyme product is reducible or oxidizeable and the measurement solution is an aqueous solution having a buffer and a salt. Preferably, the buffer is a phosphate-citrate buffer and the salt is sodium chloride.

Alternatively, the solid enzyme product is a conductive material. Preferably, the conductive material is polypyrrole. Preferably, the electrical signal comprises a difference response, wherein the difference response is a measure of the difference between a product response and a base response, wherein the product response is measured on the sites having enzyme and the base response is measured on sites not having enzyme, wherein the product response and the base response are selected from the group consisting of current, voltage, conductivity, and resistance, wherein the difference response is a measure of binding of the analytes to the capture complexes.

Preferably, the measurement time is approximately 0.1 milliseconds to 1 second.

### Brief Description of the Several Views of the Drawings

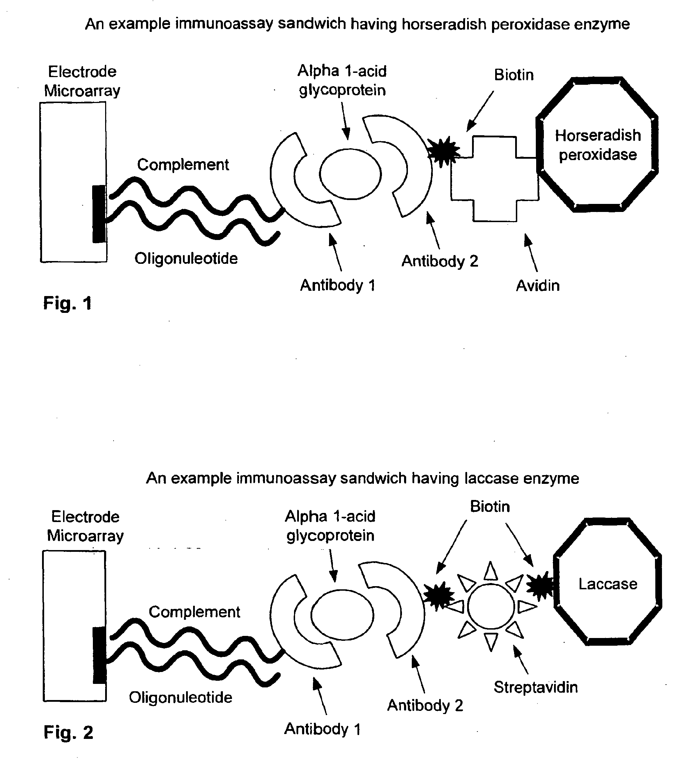

Figure 1 provides a chemical reaction scheme of the present invention when horseradish peroxidase (HRP) is used as an enzyme for detection of a binding event on a microarray. Specifically, the analyte (target) is alpha 1-acid glycoprotein (AGP), and the binding of the analyte to the microarray is detected by first forming a complex with a second antibody that is labeled with biotin. The second antibody is specific for an epitope of AGP. An avidin-labeled HRP enzyme is then added, and the avidin attaches to the biotin. The microarray site used for detecting AGP as the analyte has another antibody binding to a different epitope on AGP as the probe. The first antibody (labeled "Antibody 1") is self-assembled to an oligonucleotide microarray through a tagged probe oligonucleotide. Products from HRP catalysis are reducible and hence detectable at an electrode on a microarray by making the electrode having the HRP bound thereto a cathode.
Figure 2 provides a similar immunoassay sandwich configuration, as compared to the configuration of Figure 1, for detecting AGP at a known site on a microarray. The differences are that streptavidin is attached to a biotin-labeled second antibody that is attached to a second epitope on AGP and then a biotin-label enzyme is attached to the streptavidin. Additionally, the enzyme is laccase instead of HRP.

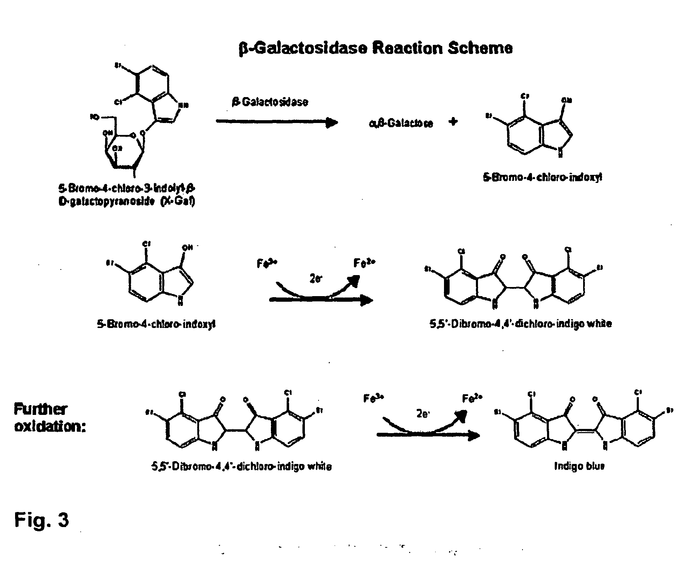

Figure 3 provides the chemistry of the enzyme reaction when beta-galactosidase is the enzyme and X-Gal (5-bromo-4-chloro-3-indoyl-beta-D-ganactopyranoside) is the substrate. The reaction forms indigo blue and generates electrochemical reactants. The reaction was carried out at approximately pH 7.0 in 0.01 molar phosphate buffered saline (PBS) with one millimolar Fe²⁺/Fe³⁺ (added as potassium ferrous cyanide and potassium ferric cyanide.) The amount of X-Gal in solution was at the saturation limit. Iron (III) functions as an electron transfer mediator.

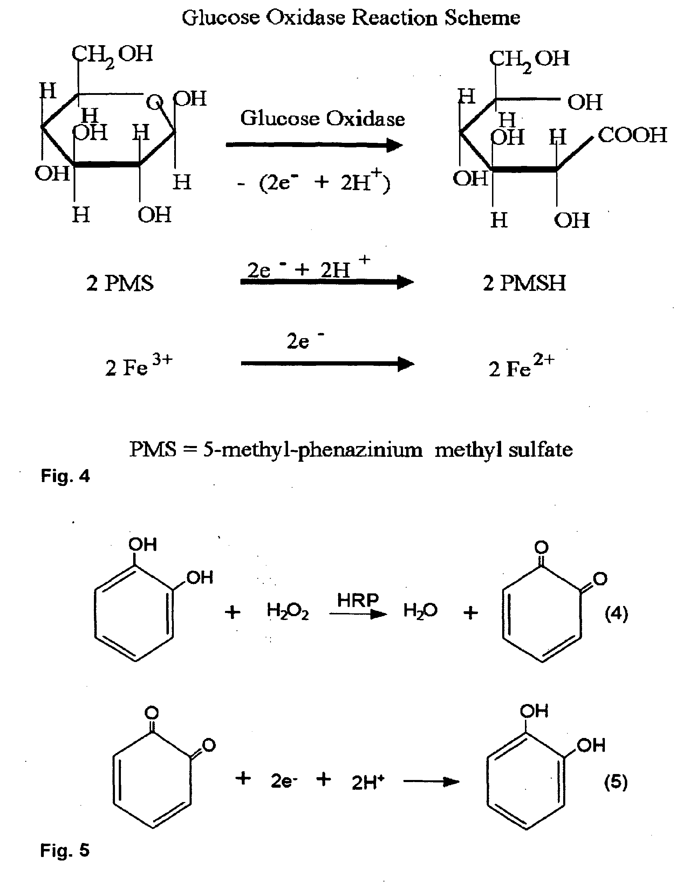

Figure 4 provides the chemistry of the enzyme reaction when glucose oxidase is the enzyme and phenazine methosulfate (5-methyl-phenazinium methyl sulfate) is the substrate. The reaction was carried out at approximately pH 7.0 in 0.01 molar phosphate buffered saline with one millimolar Fe²⁺/Fe³⁺ (added as potassium ferrous cyanide and potassium ferric cyanide.) Iron (III) functions as an electron transfer mediator.
Figure 5 provides the chemistry of the enzyme reaction when HRP is used as the enzyme and catechol and hydrogen peroxide are used as the substrates. The product of the enzyme reaction is reducible and is detected by application of a cathodic current to the electrode.

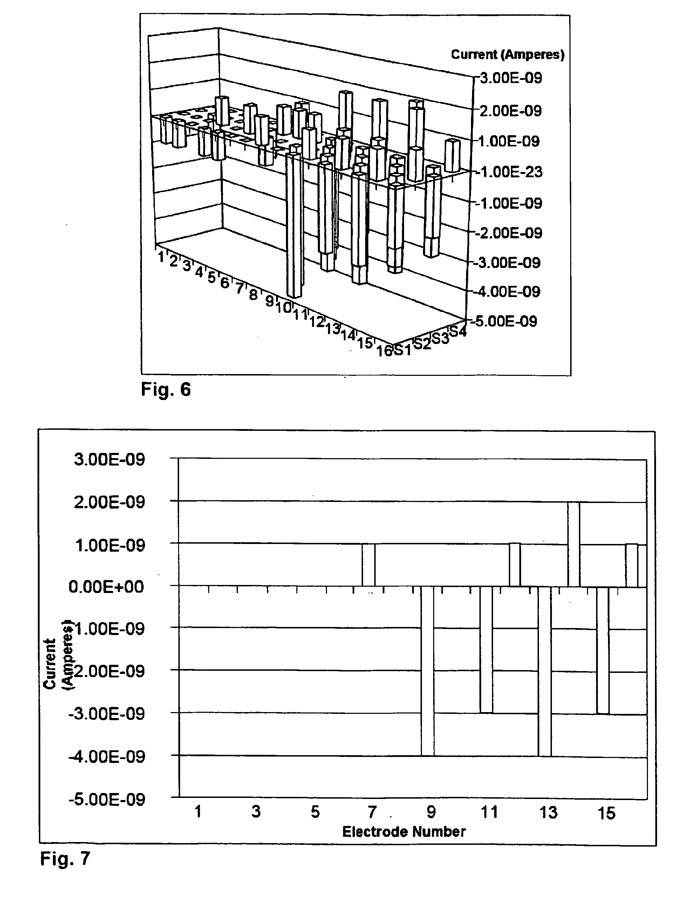

Figure 6 provides a three-dimensional plot of current measured at selected electrodes on an electrode microarray. Selected electrodes have an enzyme-amplification system using beta-galactosidase as the enzyme. The electrodes having the enzyme-amplification system were electrochemically modified using a biotin-containing reagent, which was a phosphoramidite having a biotin tag. In one section of the microarray represented by lanes 1-8, no biotin tag was placed. Lanes 9-16 show current at electrodes having the biotin tag; a checkerboard pattern was used and is shown in the figure. To attach the enzyme, Streptavidin was bound to the biotin tag, and biotinylated beta-galactosidase was bound to the streptavidin creating a biotin-streptavidin-biotin complex. Redox reagents and substrates were added according to the reaction scheme in Figure 3. The data indicate that current is higher in absolute value on electrodes having the beta-galactosidase bound thereto as shown by the negative current values in the checkerboard pattern.
Figure 7 provides a portion of the data from Figure 6. The electrodes 9, 11, 13, and 15 have beta-galactosidase bound at a site having an electrode. The current at these electrodes is negative, which indicates that a reducible enzyme product is present at the electrodes having the enzyme bound thereto.

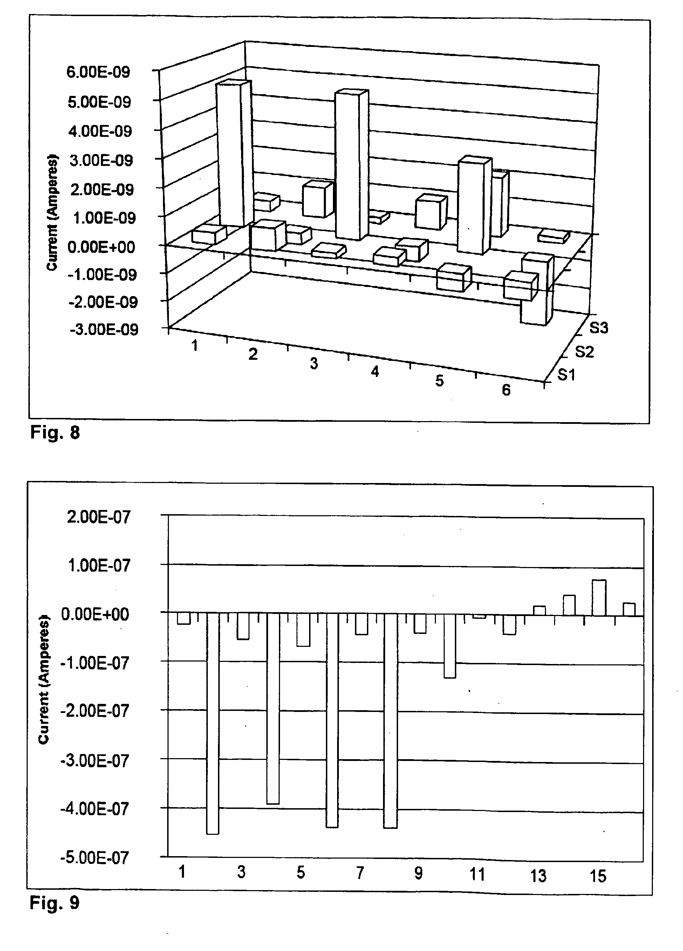

Figure 8 provides a plot of current at selected electrodes of an electrode microarray. The sign of the current was changed in this figure. Electrodes shown in lanes 1, 3, and 5 indicate that there was binding and detection of a fluorescein-labeled beta-galactosidase at those electrodes. The fluorescein acted as a low molecular weight antigen attached to the larger enzyme. The enzyme attachment was accomplished by placement of an affinity-tagged anti-fluorescein antibody on alternating electrodes in row S2. The data provides that those known locations associated with electrodes having fluorescein-labeled beta-galactosidase had higher current. The data was verified using epi-fluorescent microscopy to confirm the electrochemical results.
Figure 9 provides a plot of current at selected electrodes of an electrode microarray. The enzyme used was glucose oxidase. The glucose oxidase was bound to the microarray using a biotin-streptavidin complex. Electrodes specific affinity tags are located in lanes 2, 4, 6, 8. The glucose oxidase systems had higher background signals than other oxidation/reduction enzymes.

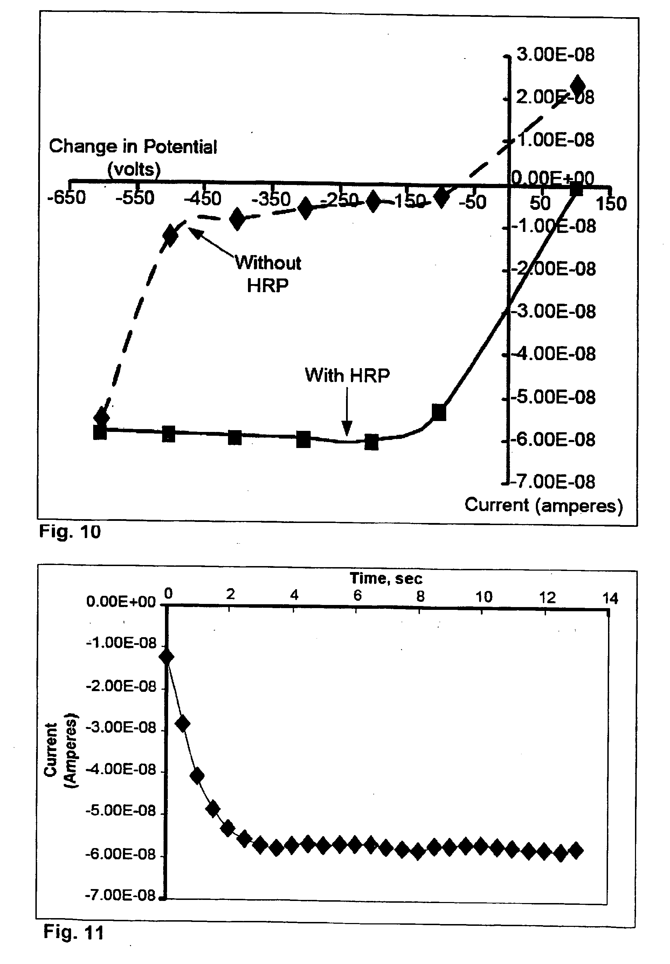

Figure 10 provides a plot of a voltage versus current for HRP as a redox curve monitored by a single 100-micrometer diameter electrode on a bare (*i.e*., no *in situ* synthesis of biomolecules) electrode microarray. The results of potentiostatic measurement indicate a negative potential, and the current difference (with and without enzyme) was substantial. Catechol and hydrogen peroxide were the substrates.
Figure 11 provides a plot of current versus time on selected electrodes of an electrode microarray. Each data point is a measurement on a different electrode. The enzyme used was HRP, and the substrates were catechol and hydrogen peroxide. The current reached a steady value after approximately three seconds.

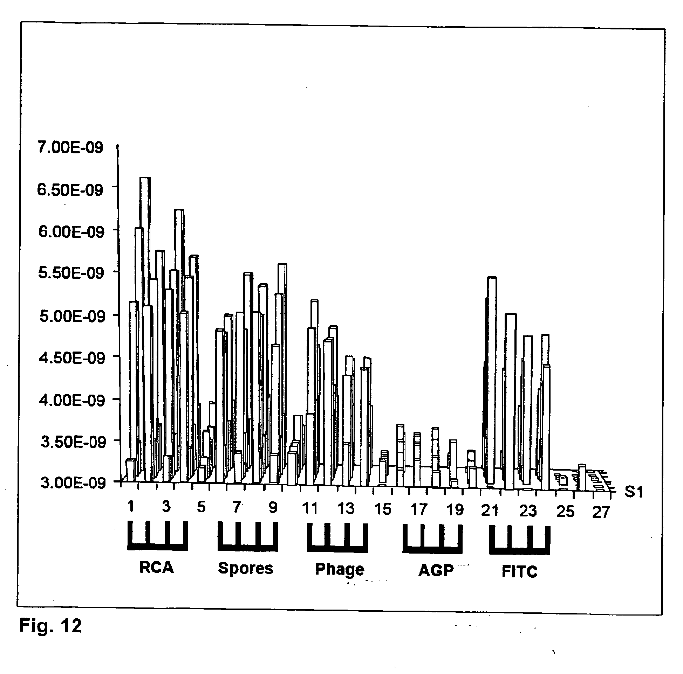

Figure 12 provides a plot of current of selected electrodes of an electrode microarray. The microarray had different analytes attached thereto in an antibody sandwich having an enzyme attached thereto. The analytes included ricin (RCA), BG spores (Bacillus globibii), Phage, AGP, and FITC. Different single stranded DNA units were synthesized for each analyte on the microarray using electrochemical synthesis. Complementary DNA strands having antibodies attached thereto for each respective analyte were hybridized to the microarray. The analytes were bound to the respective antibodies, and a second antibody was bound to each respective antibody. The second antibody had a biotin attached thereto. A complex of HRP and streptavidin was attached to the biotin. The substrates for detection were orthophenylamine diamine (ODP) and hydrogen peroxide.

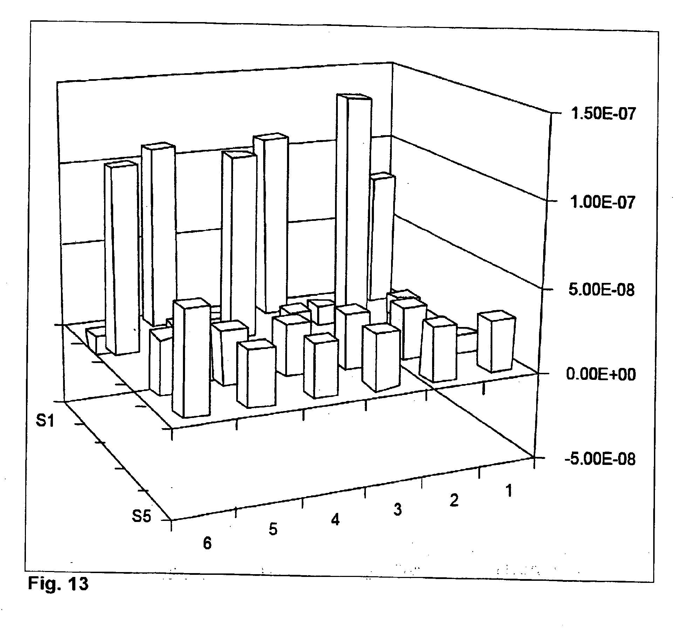

Figure 13 provides a plot of current at selected electrodes of an electrode microarray. Goat IgG was bound to electrodes S1 (1,3 and 5) and S2 (2, 4 and 6) through oligonucleotide tagging. Detection of goat IgG was made by a complex of mouse anti-goat antibody conjugated with HRP. ODP and hydrogen peroxide were used as substrates. The sign of the current is changed to make it positive.

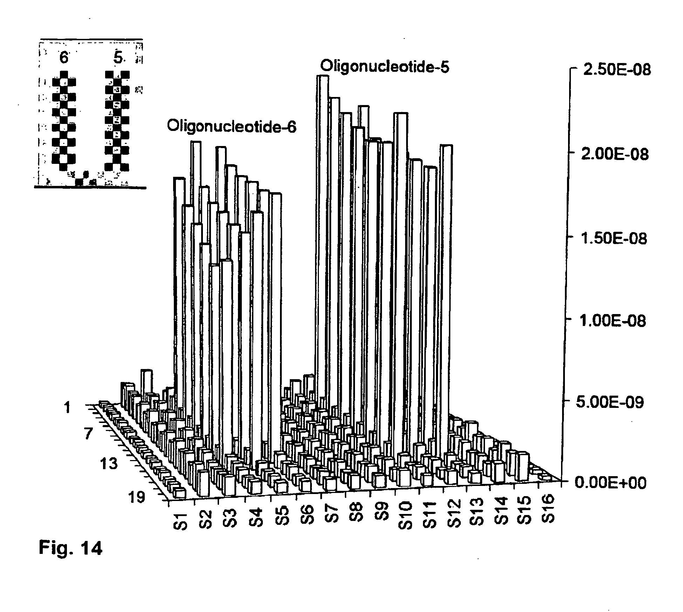

Figure 14 a plot of current of selected electrodes of an electrode microarray. Two different oligonucleotide sequences were synthesized *in situ* on the microarray. A checkerboard pattern of synthesis was used for each oligonucleotide. The checkerboard area of electrodes for each oligonucleotide comprised electrodes having the respective oligonucleotide and electrodes having no synthesis of oligonucleotide. Biotinylated target oligonucleotide samples were obtained from Operon. The biotinylated oligonucleotides were hybridized to the oligonucleotides synthesized on the microarray. Streptavidin conjugated with HRP (obtained from Sigma) was added to the microarray and bound to biotinylated oligonucleotide sequence hybridized to the microarray. Current was measured at each electrode. A schematic of the checkerboard pattern for each oligonucleotide (oligonucleotide-5 and oligonucleotide-6) is shown in the figure. The current at the electrodes is show as a three-dimensional plot in the figure. There is no signal for rabbit sequence probes. HRP was the enzyme, and the substrates for detection were orthophenylamine diamine (ODP) and hydrogen peroxide.

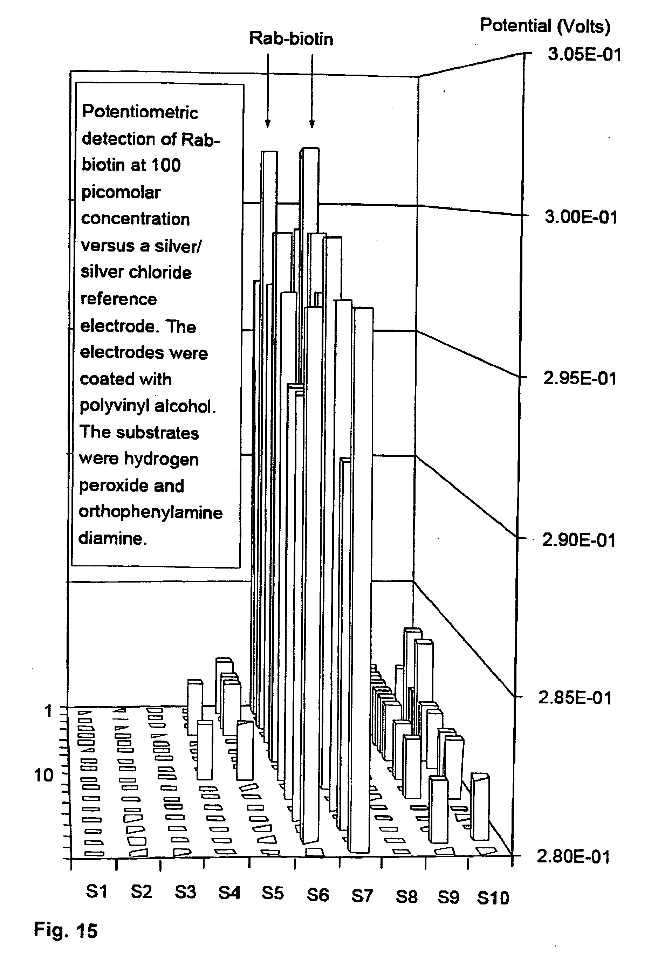

Figure 15 shows the detection of Rab-biotin at a 100 pM concentration versus a silver/silver chloride reference electrode. Detection was measured with electrical potential instead of current. HRP was the enzyme, and the substrates for detection were orthophenylamine diamine (ODP) and hydrogen peroxide.

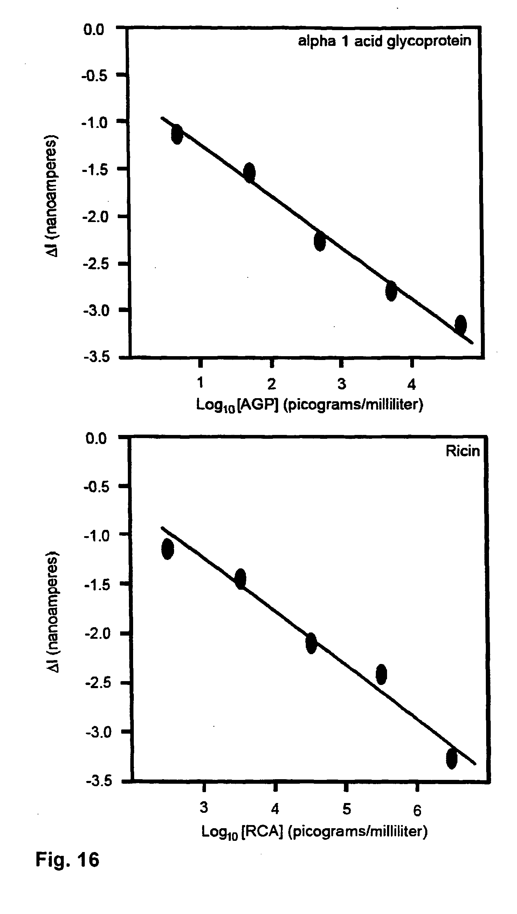

Figure 16 shows calibration curves for detecting AGP (top) and RCA (bottom). The log plots indicate high sensitivity of the present invention.

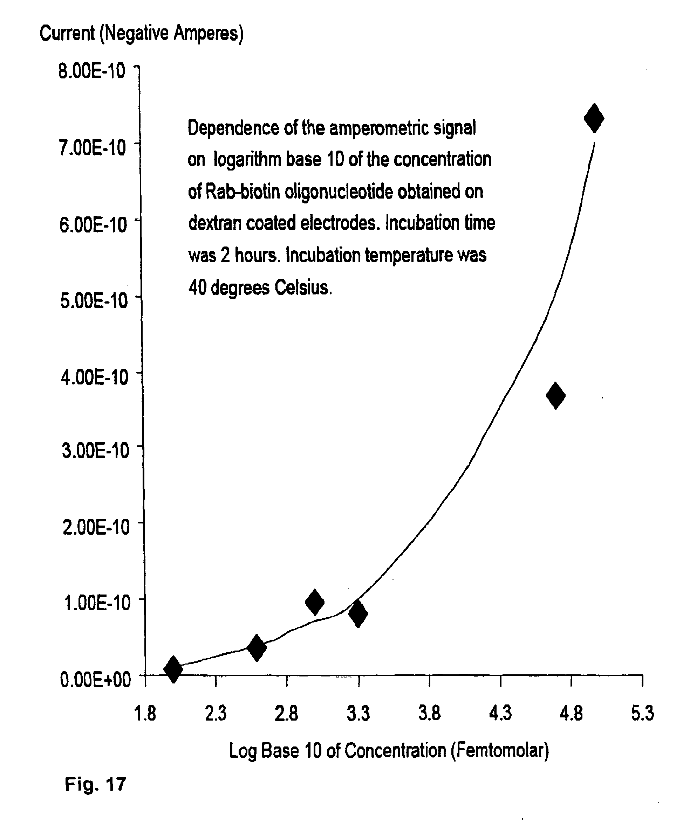

Figure 17 shows the dependence of current on the concentration of a Rab-biotin oligonucleotide. Incubation was 2 hours at 40 °C.

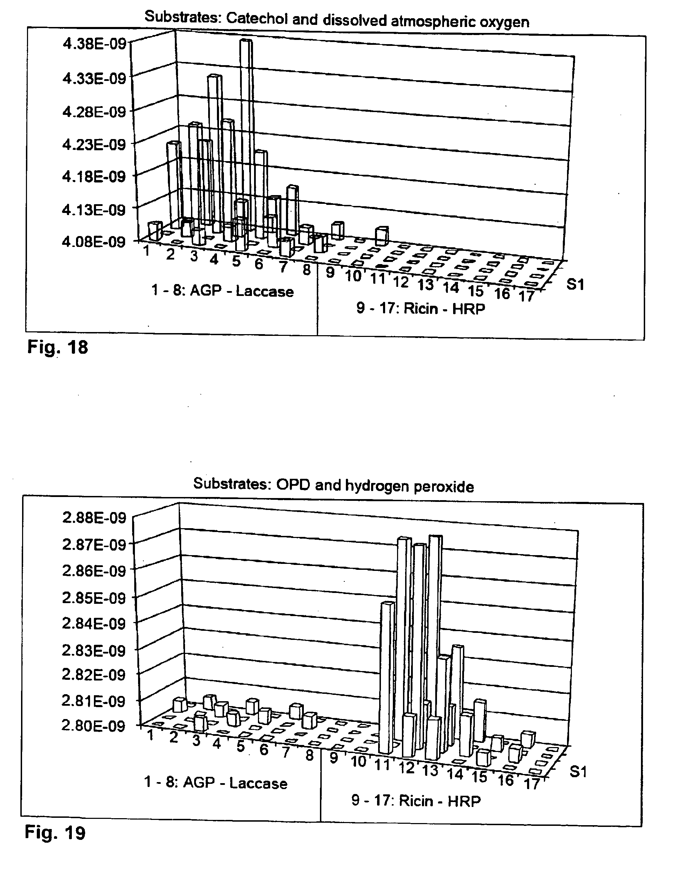

Figure 18 shows the current response of electrodes on an electrode microarray having two different enzyme systems for detection of the binding of AGP and ricin on different electrodes specific for each analyte. Shown is the response on the electrodes designed to capture AGP.
Figure 19 shows the current response of electrodes on an electrode microarray having two different enzyme systems for detection of the binding of AGP and Ricin on different electrodes specific for each analyte. Shown is the response on the electrodes designed to capture ricin.

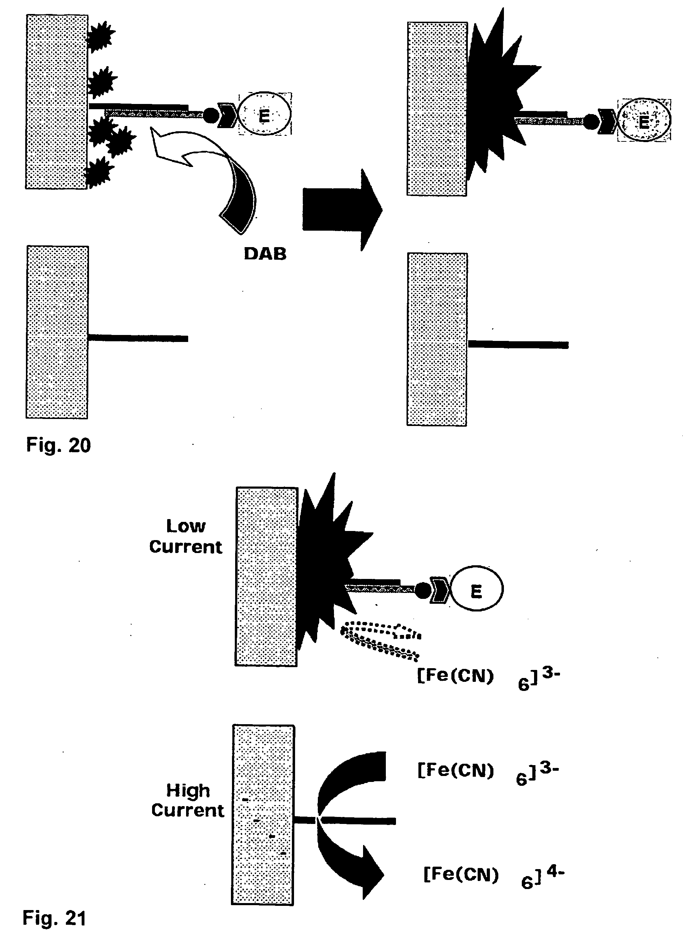

Figure 20 is a schematic representation of an electrode microarray having an electrode with an enzymatic moiety bound thereto and an electrode not having the enzymatic moiety. The enzyme reaction makes a product that is deposited at the electrode having the enzymatic moiety.
Figure 21 is a schematic representation of an electrode microarray showing the measurement of resistance at electrodes with and without a deposit. A current mediator is shown as the method for obtaining electrochemical readout of the electrodes. The mediator is shown as ferri/ferrocynanide, which is sensitive to the amount of the insoluble deposit.

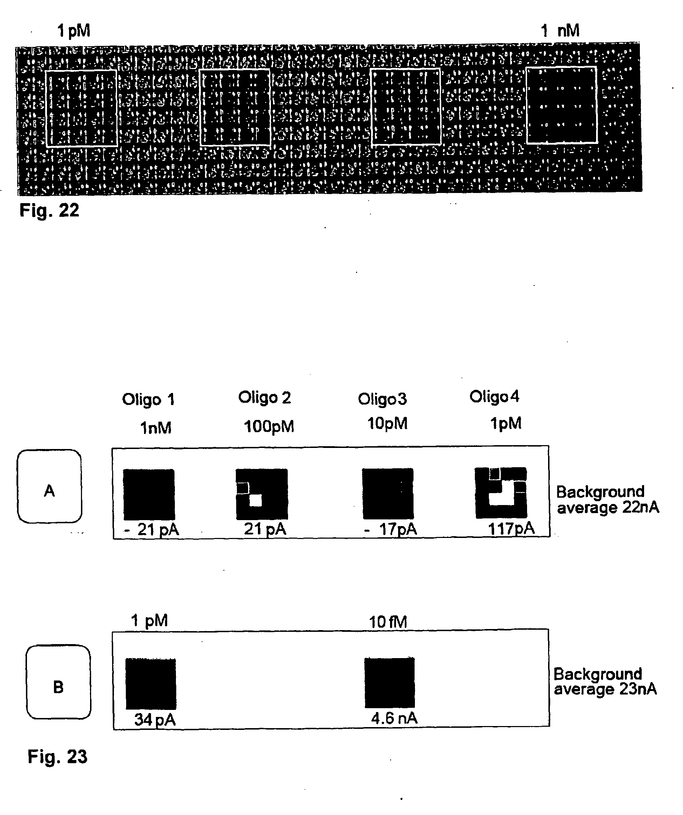

Figure 22 is a light photomicrograph of an electrode microarray having insoluble enzyme product deposited on selected electrodes. The concentration of the complementary oligonucleotide target was varied from 1 picomolar up to 1 nanomolar. The difference in the deposition of the insoluble material at high and low concentration of captured biotin-containing complementary oligonucleotides can be observed.
Figure 23 shows an electrode microarray displaying electrochemical reduction of 2 millimolar K₃[Fe(CN)₆]/K₄[Fe(CN)₆] at -100 mV on a HRP-DAB-deposit after a 30 minute deposition time.

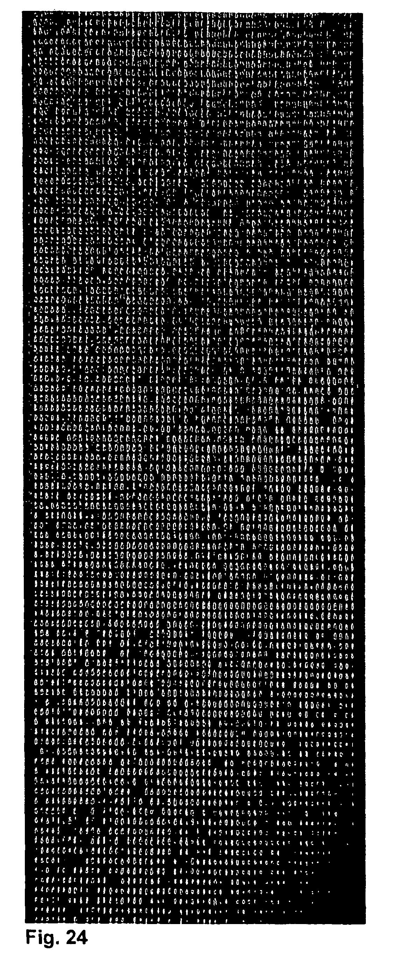

Figure 24 is an image of the electrode microarray displaying the blue deposition product as the darker electrodes. The dark blue areas indicated a high concentration of bound RNA and subsequent HRP; in contrast, the light blue indicated lower concentrations of both.

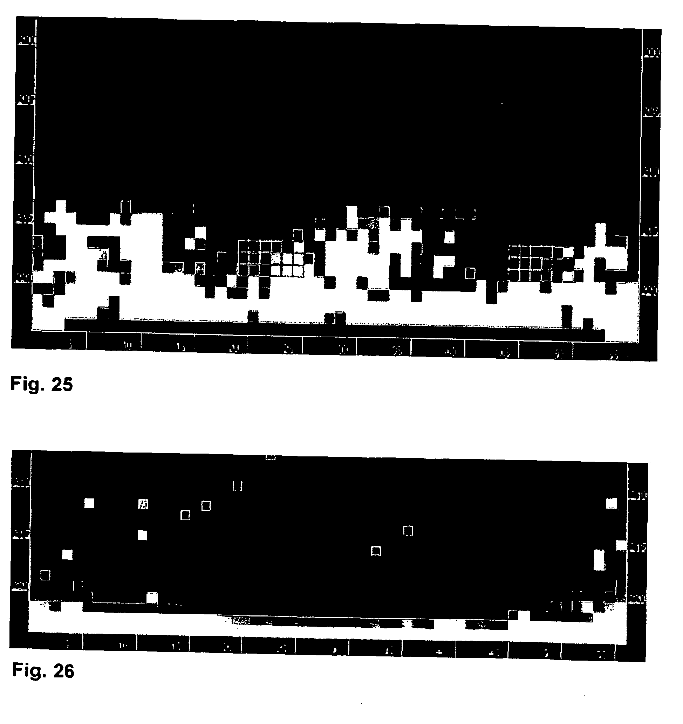

Figure 25 is an image of an electrode microarray showing a depiction of the current at each electrode during the reading of the microarray.
Figure 26 is an image of an electrode microarray showing a depiction of the current at each electrode during the reading of the microarray after all the deposited enzyme product had been removed by oxidation at the electrode. The microarray was read at -0.1 Volts. Since all of the oxidized TMB was reduced during a previous reading of the microarray, the observed current is due only to charging of the electrode. This current, on average, was 0.2-0.3 nanoamperes.

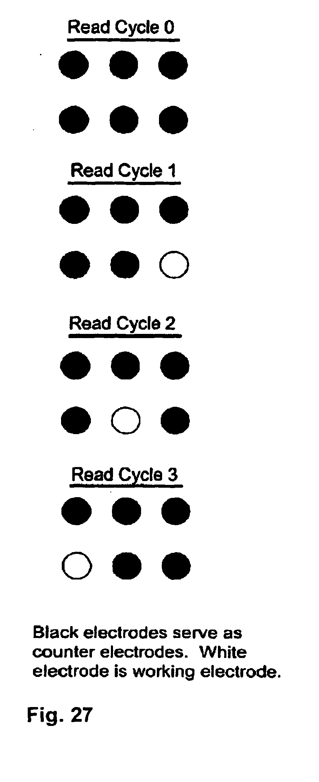

Figure 27 is a method used to read the electrical response of each electrode on an entire microarray.

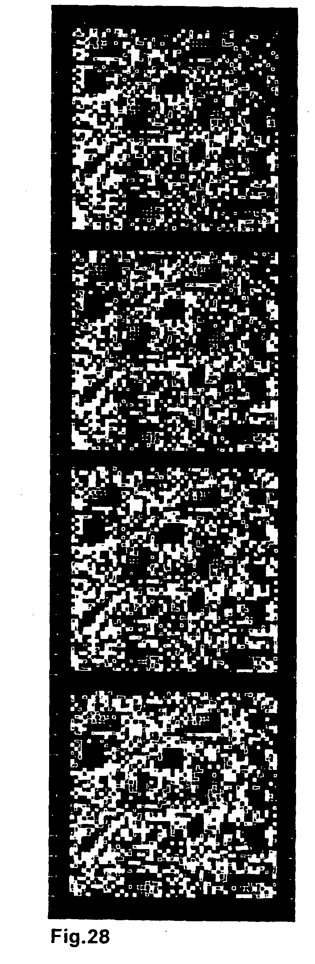

Figure 28 is an image of an electrode microarray showing a depiction of the current at each electrode during the reading of the microarray using the reading scheme of Figure 27. The white areas correspond to electrodes having probe DNA and hence faradaic current owing to the reduction of the deposition product. The black areas represent not having the deposition product and hence minimal current flow.

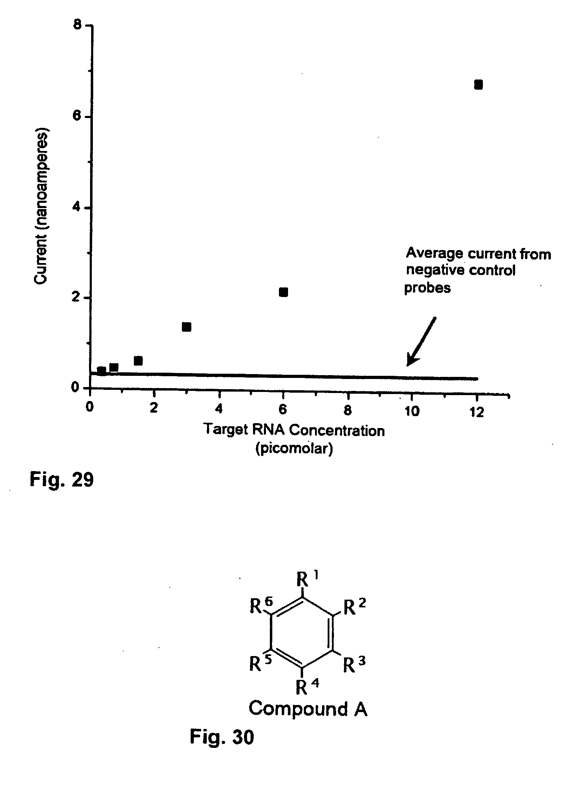

Figure 29 is a plot of current versus the concentration of complementary target RNA in solution having enzyme bound thereto. The increase in current is roughly exponential with a corresponding linear increase in concentration of target RNA in solution.
Figure 30 provides chemical structures of enzyme substrates.

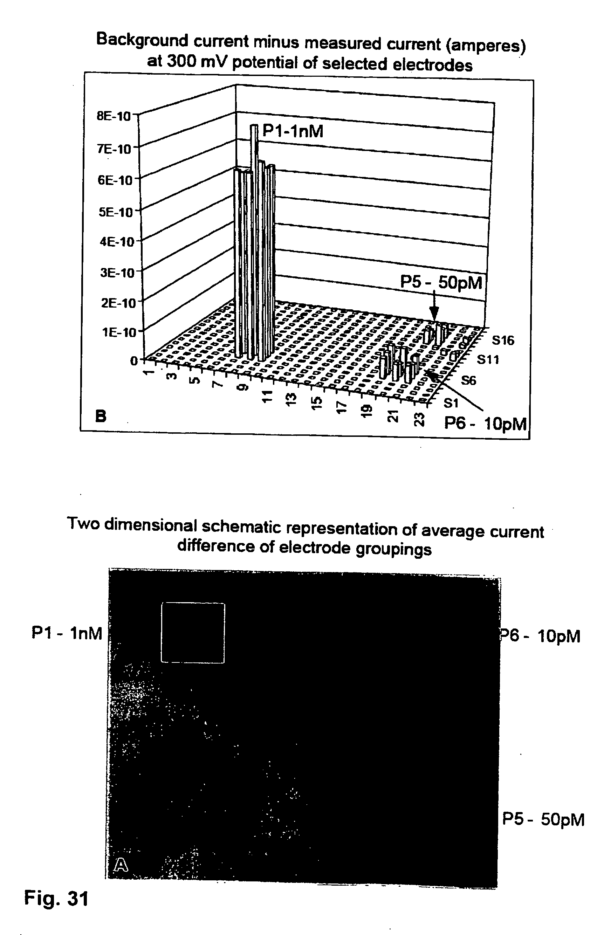

Figure 31 contains a three dimensional bar chart of current difference of electrodes having GDH and those not having GDH. Also shown is a two dimensional schematic representation of current difference of the three selected electrode groupings having GDH. The figure shows that substantial current is measured over the background current when the target oligonucleotide concentration is 1 nanomolar (P1). The current differences at 10 picomolar (P6) and 50 picomolar (P5) are less than at 1 nanomolar as expected and are approximately equal to each other. The applied voltage is 300 millivolts.

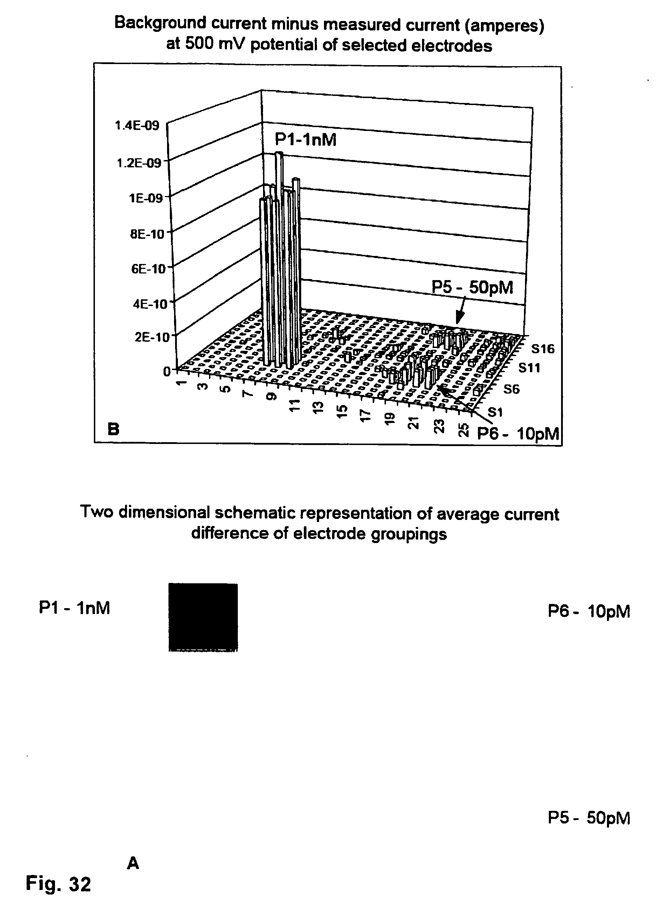

Figure 32 contains a three dimensional bar chart of current difference of electrodes having GDH and those not having GDH. Also shown is a two dimensional schematic representation of current difference of the three selected electrode groupings having GDH. The figure shows that substantial current is measured over the background current when the target oligonucleotide concentration is 1 nanomolar (P1). The current differences at 10 picomolar (P6) and 50 picomolar (P5) are less than at 1 nanomolar as expected and are approximately equal to each other. The applied voltage is 500 millivolts.

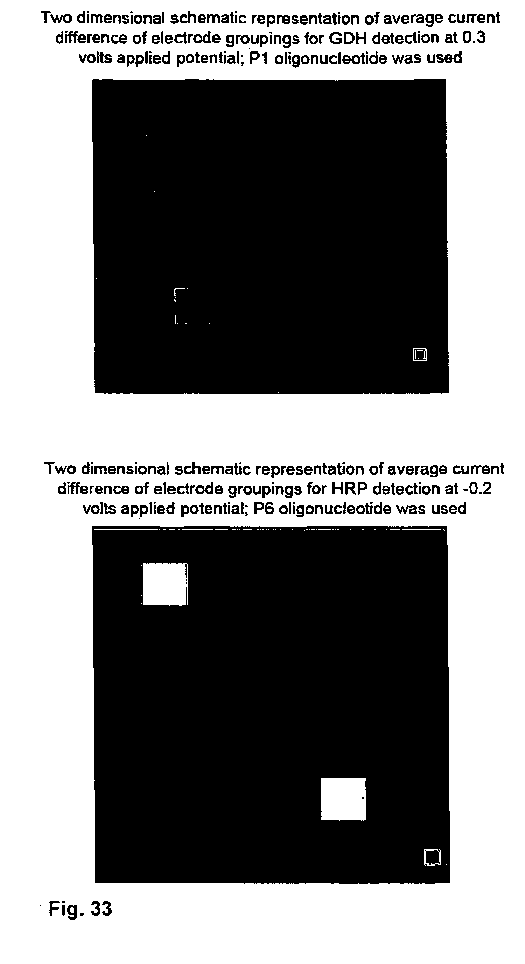

Figure 33 shows two schematic representations of average current of selected electrodes on an electrode microarray having both GDH and HRP thereon. The schematics show that during detection of either enzyme, the other enzyme is not detected, i.e., there is no crosstalk.

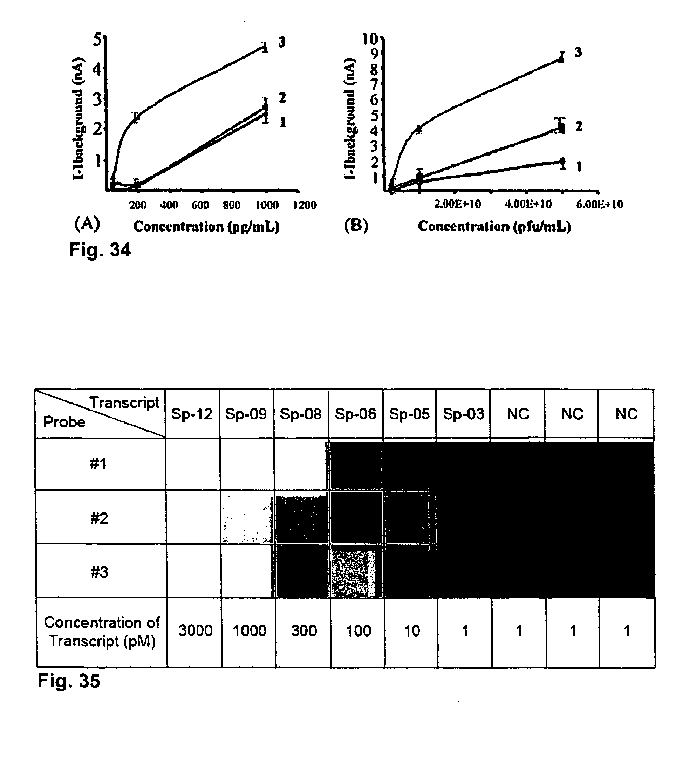

Figure 34 contains two plots of measured current minus background current as a function of concentration of two different analytes, RCA in (A) and M13 phage in (B). Incubation times of 12 minutes (curve 1), 30 minutes (curve 2), and 60 minutes (curve 3) were used.
Figure 35 provides a two dimensional depiction of the amount of current passing at different electrodes. The figure is a grayscale image of the current passing in each electrode, which is designated as a square block. A higher current corresponds to a lighter image in a block. This figure shows the three probes for each of the six transcripts hybridized (in columns per transcript) as well as the concentration in solution of the hybridized transcript (bottom row). There are also nine negative control probes that have no hybridizing species in solution.

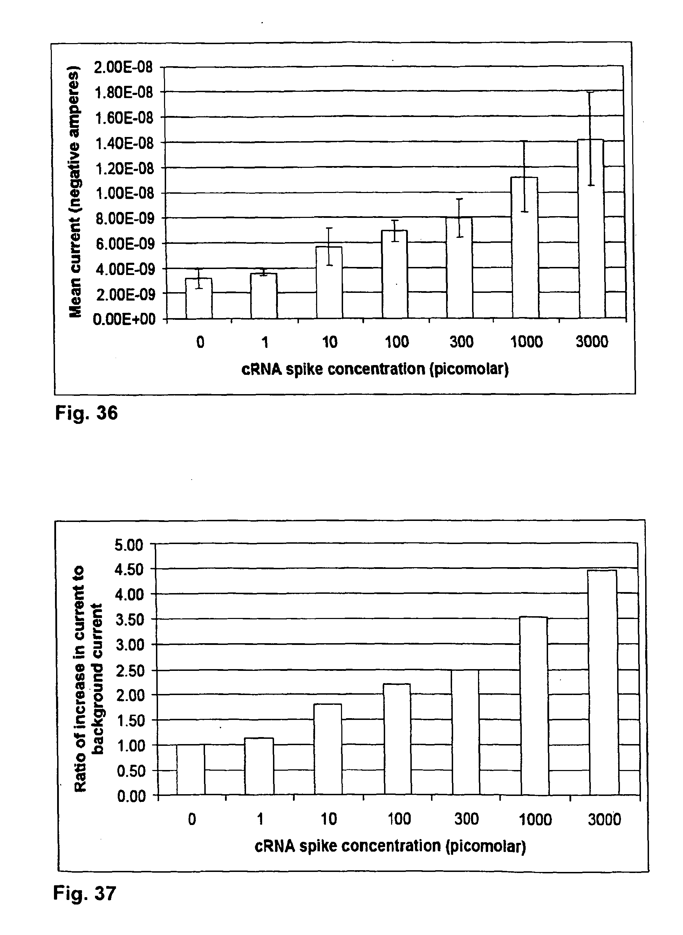

Figure 36 provides the numeric value of the current at the electrodes for each hybridized transcript. The value is the average of the three different probes for each transcript concentration.
Figure 37 provides the amount of increase relative to the average background as calculated from the nine zero concentration probes. The figure shows that current increased 1 times at the lowest concentration to 4.5 times at the highest concentration.

### Detailed Description of the Invention

The following is a definition list of terms as used herein:
The term "electrode microarray" means a microarray of electrodes on a solid substrate. Each electrode is individually addressable and can be activated as an anode or as a cathode. The solid substrate is generally planar and has a surface having the electrodes. Generally, the size of the electrodes is approximately 0.1-100µm. The number of electrodes can be as few as one and up to tens of thousands and even hundreds of thousands. Generally, the size of an electrode microarray is not limited and neither is the number of electrodes. The term "chip" means an electrode microarray. The term "microarray" includes electrode microarrays as well as other types of microarrays.
The term "reaction layer" means a coating on the electrodes, wherein the coating aids electrochemical synthesis or attachment of pre-synthesized materials. DNA, RNA, and other molecules can be electrochemically synthesized *in situ* at the electrodes of an electrode microarray or attached to the microarray electrodes. For each electrode, there are "sites" or "locations" that correspond thereto and such sites are on a part of the reaction layer. Optionally, there is an opposing surface to the electrodes having the reaction layer where synthesis or attachment occurs. The opposing surface is held close to the electrode surface and is in electrical contact by a solution that is between the electrodes and the opposing surface.
The term "probe oligonucleotide" means a single strand of DNA or RNA that usually is synthesized *in situ* on an electrode microarray although it may be spotted thereon. Probe oligonucleotides may be substantially complementary to target oligonucleotides in a solution. Probe oligonucleotided may be pre-synthesized and then attached to an electrode microarray. "Probe DNA" specifically refers to a DNA strand, and "probe RNA" specifically refers to a RNA strand.
The term "target oligonucleotide" means a single strand of DNA or RNA in a solution that may be capable of hybridizing to a probe oligonucleotide. Target oligonucleotide is not attached to the microarray other than by hybridization to probe oligonucleotide. "Target DNA" specifically refers to a DNA strand, and "target RNA" specifically refers to a RNA strand. Examples of target oligonucleotides include synthetic or natural single stranded DNA or RNA including ribosomal RNA and mitochondrial DNA. Target oligonucleotides typically have an affinity tag to allow a reporter complex to attached thereto.
The term "capture complex" means a chemical species capable of capturing an "analyte" from a solution. The analyte is a chemical species of interest, and the capturing of the analyte by the capture complex is referred to as a "binding event." Capturing generally means forming a bond, such as a hydrogen bond, but generally, does not mean forming a covalent bond. An analyte will not bind to a capture complex unless the analyte and the capture complex have affinity for each other. Affinity includes, but is not limited to, antibody-antigen interaction, streptavidin-biotin, avidin-biotin, and complementary base pairing of oligonucleotides. "Bound complex" refers to the resulting species formed by the binding event. As an example, a capture complex may comprise a probe oligonucleotide synthesized *in situ* on an electrode microarray, and a biotinylated oligonucleotide may be the analyte. The biotinylated oligonucleotide (analyte) hybridizes to the probe oligonucleotide (capture complex) when there is sufficient complementary base pairing, and the solution conditions allow hybridization. A "reporter complex" may be attached to the biotin to detect the binding event. As another example, a capture complex may comprise an antibody-target oligonucleotide compound that is hybridized to a probe oligonucleotide. The analyte is a chemical species (antigen) in a solution capable of specifically binding to the antibody. A "reporter complex" may be attached to the antigen to detect the binding event (antigen to capture complex.) A first capture complex is a complex of a specific type. Similarly, a subsequent capture complex includes any and all other capture complexes. A first analyte type is an analyte of a specific type having affinity for a first capture complex. Similarly, a subsequent capture complex includes any and all other analytes that have affinity for a specific type of capture complex. An electrode microarray may have multiple types of capture complexes for multiple targets (analytes), i.e., a first capture complex and one or more subsequent capture complexes, wherein each subsequent capture complex has affinity for a subsequent target (analyte).
The term "reporter complex" means a chemical compound that is capable of binding to a bound complex and has an enzyme as a part of the reporter complex. A reporter complex provides for a method of detecting the binding event. A reporter complex may be formed in a sequential manner by sequentially adding different chemical species that bind to each other and form the reporter complex when the last chemical species is added. The last chemical species has an enzyme. As an example, a reporter complex could be an enzyme-streptavidin complex, wherein the streptavidin is capable of binding to a biotin on a target oligonucleotide hybridized to a probe oligonucleotide. As another example, a reporter complex may comprise a "reporter antibody," which has an antibody for binding to an antigen captured by a first antibody attached to a capture complex. A reporter antibody may have a biotin bound to an antibody, a streptavidin bound to the biotin, and an enzyme bound to the streptavidin. The antibody binds to the analyte, which is bound to the first antibody. The enzyme is capable of making a redox product that is detectable at the electrode site. The reporter antibody may be formed by first adding an antibody-biotin complex followed by a streptavidin-enzyme complex. Alternatively, streptavidin may be added alone followed by a biotin-enzyme complex. Other reporter antibodies are disclosed in the specification below.
The term "buffer" means any aqueous solution having the ability to control pH or resist changes in pH. Buffers include the following compounds individually and in combination: disodium phosphate, mono-sodium phosphate, citrate, carbonate, bicarbonate, borate, acetate, 2-(N-morpholino)ethanesulfonic acid (MES), bis(2-hydroxyethyl)imino*tris*(hydroxymethyl) methane (Bis-Tris), N-(2-acetamido)-2-iminodiacetic acid (ADA), 2-[(2-amino-2-oxoethyl) amino]ethanesulfonic acid (ACES), piperaine-N,N'-*bis*(2-ethansulfonic acid (PIPES), 3-(N-morpholino)-2-hydroxypropanesulfonic acid (MOPSO), 1,3-*bis*[*tris(*hydroxymethyl) methylamino]propane (*Bis-Tris* Propane), N,N-*bis*(2-hydroxyethyl)-2-aminoethanesulfonic acid (BES), 3-(N-morpholino)propanesulfonic acid (MOPS), N-(2-hydroxyethyl)piperazine-N'-(2-ethanesulfonicacid) (HEPES), N-*tris*(hydroxymethyl)methyl-2-aminoethanesulfonic acid (TES), 3-[N,N-*bis*(2-hydroxyethyl)amino]-2-hydroxypropanesulfonic acid (DIPSO), 3-[N-*tris*(hydroxymethylamino]-2-hydroxypropanesulfonic acid (TAPSO), *tris*(hydroxymethyl) aminomethane (TRIZMA), N-(2-hydroxyethyl)piperazine-N'-(2-hydroxypropanesulfonic acid) (HEPPSO), piperazine-N,N'-*bis*(2-hydroxypropanesulfonic acid) (POPSO), N-(2-hydroxyethyl)piperazine-N'-(3-propanesulfonic acid) (EPPS), triethanolamine (TEA), N-*tris*(hydroxymethyl)methylglycine (Tricine), N,N-*bis*(2-hydroxyethyl)glycine (Bicine), N-*tris*(hydroxymethyl)methyl-3-aminopropanesulfonic acid (TAPS), 3-[(1,1-dimethyl-2-hydroxyethyl)amino]-2-hydroxypropanesulfonic acid (AMPSO), 2-(N-cyclohexylamino) ethanesulfonic acid (CHES), 3-(cyclohexylamino)-2-hydroxy-1-propanesulfonic acid (CAPSO), 2-amino-2-methyl-1-propanol (AMP), and 3-(cyclohexylamino)-1-propanesulfonic acid (CAPS).
The term "enzymatic substrate solution" means an aqueous solution having substrate molecules reactive with enzymes. Examples of substrates for different enzymes are exemplified below. Additionally, there may be any one of the following in the solution alone or in any combination: coenzymes, surfactants, preserving agents, viscosity modifiers, photostabilizers, and accelerators. Examples of coenzymes include nicotinimide adinine dinucleotide (NAD), flavin adinine dinucleotide (FAD), and hydrogen peroxide. The formulation of enzymatic substrate solutions may be proprietary to the supplier. Examples of surfactants include TWEEN®, sodium dodecylsulfate, and 3-(N-morpholino)propanesulfonic acid (MOPS). An example of a preserving agent is ethylene glycol.
The term "oxydizeable aromatics" means aromatic-based chemicals having the property of being reactive with an enzyme to make a product that is reducible at an electrode. Oxidizeable aromatics include the following compounds individually and in combination: Compound A in Figure 30 having groups R¹, R², R³, R⁴, R⁵, and R⁶, wherein R¹ is selected from the group consisting of hydroxyl, amino, -OR⁷, and -NR⁸R⁹, wherein R², R³, R⁴, R⁵, and R⁶ are independently selected from the group consisting of hydrogen, alkyl, alkenyl, alkynyl, aryl, heteroaryl, substituted alkyl, substituted alkenyl, substituted alkynyl, substituted aryl, nitrile, halogen, hydroxyl, amino, -OR⁷, -NR⁸R⁹, ester, amide, carbonyl, carbamate, urea, thio ester, thio amide, thio carbonyl, thiocarbomate, aldahyde, ketone, imine, alkene, polycyclic systems, and substituted polycyclic systems, wherein R⁷ is selected from the group consisting of alkyl, aryl, hydrogen, heteroaryl, substituted alkyl, substituted aryl, ester, ketone, aldehyde, carbamate, carbonyl, silyl ether, wherein R⁸ and R⁹ are independently selected from the group consisting of alkyl, aryl, hydrogen, heteroaryl, substituted alkyl, substituted aryl, amide, ketone, aldehyde, urea, carbamate, thio ester, thio amide, thio carbonyl, and thio carbamate.
The term "ferrocene derivatives" means chemicals having the property of being reactive with an enzyme to make a product that is reducible at an electrode. In general, ferrocene compounds are organometallic reagents having high electron transfer properties. Ferrocene derivatives include the following compounds individually and in combination: Ferrocene, Acetylferrocene, Benzoylferrocene, n-Butylferrocene, Ferrocene Monocarboxylic Acid, Butyryl Ferrocene, Cyclohexenyl Ferrocene, Cyclopentenyl Ferrocene, Dimethylaminomethyl Ferrocene, Ethyl Ferrocene, Hexanoyl Ferrocene, Hexyl Ferrocene, Octanoyl Ferrocene, Pentanoyl Ferrocene, Pentyl Ferrocene, Propionyl Ferrocene, Propyl Ferrocene, 1,1'-Diacetyl Ferrocene, 1,1'-Dibutyl Ferrocene, 1,1'-Dibutyryl Ferrocene, 1,1'-Diethyl Ferrocene, 1,1'-Dihexanoyl Ferrocene, 1,1'-Dihexyl Ferrocene, 1,1'-Dipropyl Ferrocene, 4-Ferrocenoyl Butyric Acid, 4-Ferrocenoyl Butyric Acid Esters, 3-Ferrocenoyl Propionic Acid, 3-Ferrocenoyl Propionic Acid Esters, Ferrocenyl Acetic Acid, Ferrocenyl Acetonitrile, 4-Ferrocenyl Butyric Acid, 4-Ferrocenyl Butyric Acid Esters, Ferrocenyl Carboxaldehyde, Ferroenyl Carboxylic Acid, Ferrocenyl Ethanol, Ferrocenyl Methanol, 5-Ferrocenyl Valeric Acid, 5-Ferrocenyl Valeric Acid Esters, Catocene (2,2-Bis(ethylferrocenyl)propane, aminoferrocene, formamidoferrocene, isocyanoferrocene, isothiocyanatoferrocene, and diphosphine 1,1'bis(diphenylphosphino)ferrocene. Examples of other organometallics include bis(2,2'-bipyridine)-4,4'-dicarboxybipyridine-ruthenium and zinc porphyrin.
The term "oxidizeable inorganic compounds" means inorganic chemicals having the property of being reactive with an enzyme to make a product that is reducible at an electrode. Oxidizeable inorganic compounds include the following compounds individually and in combination iodine, Mohr's salt (ammonium iron(II) sulfate), and potassium cyanoferrate(II).
The term "alkyl" means a straight or branched chain alkyl group having a single radical and containing up to approximately 20 but preferably up to 10 carbon atoms. Examples of alkyl groups include but are not limited to the following: methyl, ethyl, n-propyl, isopropyl, n-butyl, isobutyl, *sec*-butyl, *tert-butyl,* n-pentyl, isopentyl, neopentyl, *tert*-pentyl, isohexyl, n-hexyl, n-heptyl, and n-octyl. A substituted alkyl has one or more hydrogen atoms substituted by other groups or one or more carbons replaced by a divalent or trivalent group or atom.
The term "alkenyl" means a straight or branched chain alkyl group having a single radical, having at least one carbon-carbon double bond, and containing up to approximately 20 but preferably up to 10 carbon atoms. Examples of alkenyl groups include but are not limited to the following: vinyl, 1-propenyl, 2-butenyl, 1,3-butadienyl, 2-pentenyl, 2,4-hexadienyl, 4-(ethyl)-1,3-hexadienyl, and 2-(methyl)-3-(propyl)-1,3-butadienyl. A substituted alkenyl has one or more hydrogen atoms substituted by other groups or one or more carbons replaced by a divalent, trivalent, or tetravalent group or atom.
The term "alkynyl" means a straight or branched chain alkyl group having a single radical, having at least one carbon-carbon triple bond, and containing up to approximately 20 but preferably up to 10 carbon atoms. Examples of alkynyl groups include but are not limited to the following: ethynyl, 1-propynyl, 2-propynyl, 1-butynyl, 2-butynyl, 3-butynyl, 4-pentynyl, 5-hexynyl, 6-heptynyl, 7-octynyl, 1-methyl-2-butynyl, 2-methyl-3-pentynyl, 4-ethyl-2-pentynyl, and 5,5-methyl-1,3-hexynyl. A substituted alkynyl has one or more hydrogen atoms substituted by other groups or one or more carbons replaced by a divalent, trivalent, or tetravalent group or atom.
The term "cycloalkyl" means an alkyl group forming at least one ring, wherein the ring has approximately 3 to 14 carbon atoms. Examples of cycloalkyl groups include but are not limited to the following: cyclopropyl, cyclobutyl, cyclopentyl, and cyclohexyl. A substituted cycloalkyl has one or more hydrogen atoms substituted by other groups or one or more carbons replaced by a divalent, trivalent, or tetravalent group or atom.
The term "cycloalkenyl" means an alkenyl group forming at least one ring and having at least one carbon-carbon double bond within the ring, wherein the ring has approximately 3 to 14 carbon atoms. Examples of cycloalkenyl groups include but are not limited to the following: cyclopropenyl, cyclobutenyl, cyclopentenyl, 1,3-cyclopentadienyl, and cyclohexenyl. A substituted cycloalkenyl has one or more hydrogen atoms substituted by other groups or one or more carbons replaced by a divalent, trivalent, or tetravalent group or atom.
The term "cycloalkynyl" means an alkynyl group forming at least one ring and having at least one carbon-carbon triple bond, wherein the ring contains up to approximately 14 carbon atoms. A group forming a ring having at least one triple bond and having at least one double bond is a cycloalkynyl group. An example of a cycloalkynyl group includes but is not limited to cyclooctyne. A substituted cycloalkynyl has one or more hydrogens substituted by other groups or one or more carbons replaced by a divalent, trivalent, or tetravalent group or atom.
The term "aryl" means an aromatic ring group having mostly carbon atoms and a single radical and having approximately 4 to 20 carbon atoms. An aryl ring structure can include a ring with one or two heteroatoms. Examples of aryl groups include but are not limited to the following: phenyl, naphthyl, and anthryl. A substituted aryl has one or more hydrogens substituted by other groups or one or more carbons replaced by a divalent or trivalent group or atom.
The term "hetero," when used in the context of chemical groups, or "heteroatom" means an atom other than carbon or hydrogen. Examples of heteroatoms include but are not limited to the following: oxygen, nitrogen, phosphorous, sulfur, boron, silicon, and selenium.
The term "heterocyclic ring" means a ring structure having at least one ring having at least one heteroatom forming a part of the ring and having approximately 3 to 20 atoms connected to form the ring structure. An example of a heterocyclic ring having 6 atoms is pyridine. Additional examples of heterocyclic ring structures include but are not limited to the following aromatic structures: acridine, carbazole, chromene, imidazole, furan, indole, quinoline, and phosphinoline. Examples of heterocyclic ring structures include but are not limited to the following non-aromatic structures: aziridine, 1,3-dithiolane, 1,3-diazetidine, and 1,4,2-oxazaphospholidine. Examples of heterocyclic ring structures include but are not limited to the following fused aromatic and non-aromatic structures: 2*H*-furo[3,2-*b*]pyran, 5*H-*pyrido[2,3-*d*]-*o*-oxazine, 1*H*-pyrazolo[4,3-*d*]oxazole, 4*H*-imidazo[4,5-*d*]thiazole, selenazolo[5,4-*f*]benzothiazole, and cyclopenta[b]pyran.
The term "polycyclic group" means a carbon ring structure having more than one ring and having approximately 4 to 20 carbons forming the ring structure. Examples of polycyclic groups include but are not limited to the following: bicyclo[1.1.0]butane, bicyclo[5.2.0]nonane, and tricycle[5.3.1.1]dodecane.
The term "halo" or "halogen" means inclusively, fluorine, chlorine, bromine, or iodine.
The term "heteroatom group" means one heteroatom or more than one heteroatoms bound together and having two free valences for forming a covalent bridge between two atoms. For example, the oxy radical, -O- can form a bridge between two methyls to form CH₃-O-CH₃ (dimethyl ether) or can form a bridge between two carbons to form an epoxy such as cis or trans 2,3-epoxybutane. As used herein and in contrast to the normal usage, the term heteroatom group will be used to mean the replacement of groups in an alkyl, alkenyl, alkynyl, cycloalkyl, cycloalkenyl, and cycloalkynyl and not the formation of cyclic bridges, such as an epoxy, unless the term cyclic bridge is used with the term heteroatom group to denote the normal usage. Examples of heteroatom groups, using the nomenclature for hetero bridges (such as an epoxy bridge), include but are not limited to the following: azimino (-N=N-HN-), azo (-N=N-), biimino (-NH-HN-), epidioxy (-O-O-), epidithio (-S-S-), epithio (-S-), epithioximino (-S-O-NH-), epoxy (-O-), epoxyimino (-O-NH-), epoxynitrilo (-O-N=), epoxythio (-O-S-), epoxythioxy (-O-S-O-), furano (-C₄H₂O-), imino (-NH-), and nitrilo (-N=). Examples of heteroatom groups using the nomenclature for forming acyclic bridges include but are not limited to the following: epoxy (-O-), epithio (-S-), episeleno (-Se-), epidioxy (-O-O-), epidithio (-S-S-), lambda⁴-sulfano (-SH₂-), epoxythio (-O-S-), epoxythioxy (-O-S-O-), epoxyimino (-O-NH-), epimino (-NH-), diazano (-NH-HN-), diazeno (-N=N-), triaz[1]eno (-N=N-HN-), phosphano (-PH-), stannano (-SnH₂-), epoxymethano (-O-CH₂-), epoxyethano (-O-CH₂-CH₂-), epoxyprop[1]eno (-O-CH-CH-CH₂-).
The term "bridge" means a connection between one part of a ring structure to another part of the ring structure by a hydrocarbon bridge. Examples of bridges include but are not limited to the following: methano, ethano, etheno, propano, butano, 2-buteno, and benzeno.
The term "hetero bridge" means a connection between one part of a ring structure to another part of the ring structure by one or more heteroatom groups, or a ring formed by a heterobridge connecting one part of a linear structure to another part of the linear structure, thus forming a ring.
The term "oxy" means the divalent radical -O-.
The term "oxo" means the divalent radical =O.
The term "carbonyl" means the group -C(O)-, wherein the carbon has two radicals for bonding.
The term "amide" or "acylamino" means the group -NH-C(O)-R, wherein the nitrogen has one single radical for bonding and R is hydrogen or an unsubstituted or substituted alkyl, alkenyl, alkynyl, cycloalkyl, cycloalkenyl, cycloalkynyl, aryl, heterocyclic ring, or polycyclic group.
The term "alkoxy" means the group -O-R, wherein the oxygen has a single radical and R is hydrogen or an unsubstituted or substituted alkyl, alkenyl, alkynyl, cycloalkyl, cycloalkenyl, cycloalkynyl, aryl, heterocyclic ring, or polycyclic group. Examples of alkoxy groups where the R is an alkyl include but are not limited to the following: methoxy, ethoxy, propoxy, butoxy, pentoxy, hexoxy, heptoxy, octoxy, 1,1-dimethylethoxy, 1,1-dimethylpropoxy, 1,1-dimethylbutoxy, 1,1-dimethylpentoxy, 1-ethyl-1-methylbutoxy, 2,2-dimethylpropoxy, 2,2-dimethylbutoxy, 1-methyl-1-ethylpropoxy, 1,1-diethylpropoxy, 1,1,2-trimethylpropoxy, 1,1,2-trimethylbutoxy, 1,1,2,2-tetramethylpropoxy. Examples of alkoxy groups where the R is an alkenyl group include but are not limited to the following: ethenyloxy, 1-propenyloxy, 2-propenyloxy, 1-butenyloxy, 2-butenyloxy, 3-butenyloxy, 1-methyl-prop-2-enyloxy, 1,1-dimethyl-prop-2-enyloxy, 1,1,2-trimethyl-prop-2-enyloxy, and 1,1-dimethyl-but-2-enyloxy, 2-ethyl-1,3-dimethyl-but-1-enyloxy. Examples of alkyloxy groups where the R is an alkynyl include but are not limited to the following: ethynyloxy, 1-propynyloxy, 2-propynyloxy, 1-butynyloxy, 2-butynyloxy, 3-butynyloxy, 1-methyl-prop-2-ynyloxy, 1,1-dimethyl-prop-2-ynyloxy, and 1,1-dimethyl-but-2-ynyloxy, 3-ethyl-3-methyl-but-1-ynyloxy. Examples of alkoxy groups where the R is an aryl group include but are not limited to the following: phenoxy, 2-naphthyloxy, and 1-anthyloxy.
The term "acyl" means the group -C(O)-R, wherein the carbon has a single radical and R is hydrogen or an unsubstituted or substituted alkyl, alkenyl, alkynyl, cycloalkyl, cycloalkenyl, cycloalkynyl, aryl, heterocyclic ring, or polycyclic group. Examples of acyl groups include but are not limited to the following: acetyl, propionyl, butyryl, isobutyryl, valeryl, isovaleryl, acryloyl, propioloyl, mathacryloyl, crotonoyl, isocrotonoyl, benzoyl, and naphthoyl.
The term "acyloxy" means the group -O-C(O)-R, wherein the oxygen has a single radical and R is hydrogen or an unsubstituted or substituted alkyl, alkenyl, alkynyl, cycloalkyl, cycloalkenyl, cycloalkynyl, aryl, heterocyclic ring, or polycyclic group. Examples of acyloxy groups include but are not limited to the following: acetoxy, ethylcarbonyloxy, 2-propenylcarbonyloxy, pentylcarbonyloxy, 1-hexynylcarbonyloxy, benzoyloxy, cyclohexylcarbonyloxy, 2-naphthoyloxy, 3-cyclodecenylcarbonyloxy.
The term "oxycarbonyl" means the group -C(O)-O-R, wherein the carbon has a single radical and R is hydrogen or an unsubstituted or substituted alkyl, alkenyl, alkynyl, cycloalkyl, cycloalkenyl, cycloalkynyl, aryl, heterocyclic ring, or polycyclic group. Examples of oxycarbonyl groups include but are not limited methoxycarbonyl, ethoxycarbonyl, isopropyloxycarbonyl, phenoxycarbonyl, and cyclohexyloxycarbonyl.
The term "alkoxycarbonyloxy" means the group -O-C(O)-O-R, wherein the oxygen has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "carboxy" means the group -C(O)-OH, wherein the carbon has a single radical.
The term "amino" means the group -NH₂, where the nitrogen has a single radical.
The term "secondary amino" means the group -NH-R, wherein the nitrogen has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "tertiary amino" means the group -NR²R¹, wherein the nitrogen atom has a single radical and R₁ and R₂ are independently selected from the group consisting of unsubstituted and substituted alkyl, alkenyl, alkynyl, cycloalkyl, cycloalkenyl, cycloalkynyl, aryl, heterocyclic ring, and polycyclic group.
The term "hydrazi" means the group -NH-NH-, wherein the nitrogens have single radicals bound to the same atom. The term "hydrazo" means the group -NH-NH-, wherein the nitrogen atoms have single radicals bound to the different atoms.
The term "hydrazino" means the group NH₂-N*H-, wherein the nitrogen (N*) has a single radical.
The term "hydrazono" means the group NH₂-N*=, wherein the nitrogen (N*) has two radicals.
The term "hydroxyimino" means the group HO-N*=, wherein the nitrogen (N*) has two radicals.
The term "alkoxyimino" means the group R-O-N*=, wherein the nitrogen (N*) has two radicals and R is an unsubstituted or substituted alkyl, alkenyl, alkynyl, cycloalkyl, cycloalkenyl, cycloalkynyl, aryl, heterocyclic ring, or polycyclic group.
The term "azido" means the group N₃-, wherein the nitrogen (N*) has one radical.
The term "azoxy" means the group -N*(O)=N*-, wherein the nitrogens each have one radical.
The term "alkazoxy" means the group R-N(O)=N*-, wherein the nitrogen (N*) has one radical and R is hydrogen or an unsubstituted or substituted alkyl, alkenyl, alkynyl, cycloalkyl, cycloalkenyl, cycloalkynyl, aryl, heterocyclic ring, or polycyclic group. Azoxybenzene is an example compound.
The term "cyano" means the group -CN. The term "isocyano" means the group -NC. The term "cyanato" means the group -OCN. The term "isocyanato" means the group -NCO. The term "fulminato" means the group -ONC. The term "thiocyanato" means the group -SCN. The term "isothiocyanato" means the group -NCS. The term "selenocyanato" means the group -SeCN. The term "isoselenocyanato" means the group-NCSe.
The term "carboxyamido" or "acylamino" means the group -NH-CO-R, wherein the nitrogen has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "acylimino" means the group =N-C(O)-R, wherein the nitrogen has two radicals and R is hydrogen or an unsubstituted or substituted alkyl, alkenyl, alkynyl, cycloalkyl, cycloalkenyl, cycloalkynyl, aryl, heterocyclic ring, or polycyclic group.
The term "nitroso" means the group O=N-, wherein the nitrogen has a single radical.
The term "aminooxy" means the group -O-NH₂, wherein the oxygen has a single radical.
The term "carxoimidioy" means the group -C(NH)-R, wherein the carbon has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "hydrazonoyl" means the group -C(NH-NH₂)-R, wherein the carbon has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "hydroximoyl" or "oxime" means the group -C(NH-OH)-R, wherein the carbon has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "hydrazino" means the group R-C(O)-NH-HN-, wherein each nitrogen has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "amidino" means the group -C(NH)-NH₂, wherein the carbon has a single radical.
The term "sulfide" means the group -S-R, wherein the sulfur has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "thiol" means the group -S-, wherein the sulfur has two radicals. Hydrothiol means -SH.
The term "thioacyl" means the group -C(S)-R, wherein the carbon has a single radical and R is hydrogen or an unsubstituted or substituted alkyl, alkenyl, alkynyl, cycloalkyl, cycloalkenyl, cycloalkynyl, aryl, heterocyclic ring, or polycyclic group.
The term "sulfoxide" means the group -S(O)-R, wherein the sulfur has a single radical and R is hydrogen or an unsubstituted or substituted alkyl, alkenyl, alkynyl, cycloalkyl, cycloalkenyl, cycloalkynyl, aryl, heterocyclic ring, or polycyclic group. The term "thiosulfoxide" means the substitution of sulfur for oxygen in sulfoxide; the term includes substitution for an oxygen bound between the sulfur and the R group when the first carbon of the R group has been substituted by an oxy group and when the sulfoxide is bound to a sulfur atom on another group.
The term "sulfone" means the group -S(O)(O)-R, wherein the sulfur has a single radical and R is hydrogen or an unsubstituted or substituted alkyl, alkenyl, alkynyl, cycloalkyl, cycloalkenyl, cycloalkynyl, aryl, heterocyclic ring, or polycyclic group. The term "thiosulfone" means substitution of sulfur for oxygen in one or two locations in sulfone; the term includes substitution for an oxyen bound between the sulfur and the R group when the first carbon of the R group has been substituted by an oxy group and when the sulfone is bound to a sulfur atom on another group.
The term "phosphoric acid ester" means the group R₁R₂PO₄-, wherein the oxygen has a single radical and R₁ is selected from the group consisting of hydrogen and unsubstituted and substituted alkyl, alkenyl, alkynyl, cycloalkyl, cycloalkenyl, cycloalkynyl, aryl, heterocyclic ring, and polycyclic group, and R₂ is selected from the group consisting of unsubstituted and substituted alkyl, alkenyl, alkynyl, cycloalkyl, cycloalkenyl, cycloalkynyl, aryl, heterocyclic ring, and polycyclic group.
The term "substituted" or "substitution," in the context of a chemical species, means independently selected from the group consisting of the replacement of a hydrogen on at least one carbon by a monovalent radical, the replacement of two hydrogens on at least one carbon by a divalent radical, the replacement of three hydrogens on at least one terminal carbon (methyl group) by a trivalent radical, the replacement of at least one carbon and the associated hydrogens (*e.g*., methylene group) by a divalent, trivalent, or tetravalent radical, and combintations thereof. Meeting valence requirements restricts substitution. Substitution occurs on alkyl, alkenyl, alkynyl, cycloalkyl, cycloalkenyl, cycloalkynyl, aryl, heterocyclic ring, and polycyclic groups, providing substituted alkyl, substituted alkenyl, substituted alkynyl, substituted cycloalkyl, substituted cycloalkenyl, substituted cycloalkynyl, substituted aryl group, substituted heterocyclic ring, and substituted polycyclic groups. The groups that are substituted on an alkyl, alkenyl, alkynyl, cycloalkyl, cycloalkenyl, cycloalkynyl, aryl, heterocyclic ring, and polycyclic groups are independently selected from the group consisting of alkyl, alkenyl, alkynyl, cycloalkyl, cycloalkenyl, cycloalkynyl, aryl, heterocyclic ring, polycyclic group, halo, heteroatom group, oxy, oxo, carbonyl, amide, alkoxy, acyl, acyloxy, oxycarbonyl, alkoxycarbonyloxy, carboxy, imino, amino, secondary amino, tertiary amino, hydrazi, hydrazino, hydrazono, hydroxyimino, azido, azoxy, alkazoxy, cyano, isocyano, cyanato, isocyanato, thiocyanato, fulminato, isothiocyanato, isoselenocyanato, selenocyanato, carboxyamido, acylimino, nitroso, aminooxy, carboximidoyl, hydrazonoyl, oxime, acylhydrazino, amidino, sulfide, thiol, sulfoxide, thiosulfoxide, sulfone, thiosulfone, thiosulfate, hydroxyl, formyl, hydroxyperoxy, hydroperoxy, peroxy acid, carbamoyl, trimethyl silyl, nitrilo, nitro, aci-nitro, nitroso, semicarbazono, oxamoyl, pentazolyl, seleno, thiooxi, sulfamoyl, sulfenamoyl, sulfeno, sulfinamoyl, sulfino, sulfinyl, sulfo, sulfoamino, sulfonato, sulfonyl, sulfonyldioxy, hydrothiol, tetrazolyl, thiocarbamoyl, thiocarbazono, thiocarbodiazono, thiocarbonohydrazido, thiocarbonyl, thiocarboxy, thiocyanato, thioformyl, thioacyl, thiosemicarbazido, thiosulfino, thiosulfo, thioureido, thioxo, triazano, triazeno, triazinyl, trithio, trithiosulfo, sulfinimidic acid, sulfonimidic acid, sulfinohydrazonic acid, sulfonohydrazonic acid, sulfinohydroximic acid, sulfonohydroximic acid, and phosphoric acid ester, and combinations thereof. As an example of a substitution, replacement of one hydrogen on ethane by a hydroxyl provides ethanol, and replacement of two hydogens by an oxo on the middle carbon of propane provides acetone (dimethyl ketone.) As a further example, replacement of the middle carbon (the methenyl group) of propane by the oxy radical (-O-) provides dimethyl ether (CH₃-O-CH₃.) As a futher example, replacement of one hydrogen on benzene by a phenyl group provides biphenyl. As provided above, heteroatom groups can be substituted inside an alkyl, alkenyl, or alkylnyl group for a methylene group (:CH₂) thus forming a linear or branched substituted structure rather than a ring or can be substituted for a methylene inside of a cycloalkyl, cycloalkenyl, or cycloalkynyl ring thus forming a heterocyclic ring. As a further example, nitrilo (-N=) can be substituted on benzene for one of the carbons and associated hydrogen to provide pyridine, or oxy can be substituted to provide pyran.
The term "unsubstituted" means that no hydrogen or carbon has been replaced on an alkyl, alkenyl, alkynyl, cycloalkyl, cycloalkenyl, cycloalkynyl, or aryl group.

### Addressable Electrode Microarrays and Binding Events

Electrode microarrays comprise a plurality of addressable electrodes. An addressable electrode is one where the electrode can be electronically controlled to create a current or voltage at the electrode. Electrode microarrays are preferably in a column and row format although other formats may be used. Electrodes may be circular or other suitable geometries including a partial annulus or a grid of lines suitably broken. Other geometries may be used including those disclosed in U.S. Appl. No 11/108,078, filed 04/15/2005, entitled "Neutralization and Containment of Redox Species Produced by Circumferential Electrodes," Each electrode occupies a surface region of the microarray. Within a specific region having an electrode, molecules can be synthesized *in situ.* The types of molecules that may be synthesized include small molecules, oligomers, and polymers. Biomolecules such as peptides, DNA, and RNA may also be synthesized. Molecules synthesized on an electrode microarray are generally referred to as probes.

Electrode microarrays further and often have a porous reaction matrix (layer) attached to the microarrays surface. The porous reaction matrix has probes attached to it and provides a three dimensional virtual flask for confinement of reagent at the electrode. The flask may be thought of as cylindrical in the case of a circular electrode. In a preferred embodiment, the porous reaction matrix is selected from the group consisting of sucrose, monosaccharides, disaccharides, trisaccharides, polyethylene glycol, polyethylene glycol derivative, N-hydroxysuccinimide, succinimide derivatives, and combinations thereof In the preferred embodiment, other porous reaction matrix materials may be used including those disclosed in U.S. Appl. No. 10/992,252, filed 11/18/2004, entitled "Electrode Array device having an adsorbed porous reaction layer," In another embodiment, the porous reaction layer is a membrane, wherein the membrane material is selected from the group consisting of polyvinyl alcohol, polyvinyl acetate, tricellulose acetate, polyurethane, agarose, controlled porosity glass with a PTFE resin, and combinations thereof.

Most commonly, a microarray contains a plurality of probe molecules Alternatively and rarely, a microarray may have one probe molecule type. In the most common form of a microarray, the probes are oligonucleotides that can bind to a complementary sequence of DNA or RNA, wherein the DNA or RNA is a target oligonucleotide. Depending upon the hybridization conditions, a target having a region that is nearly complementary to a probe may bind to the probe. Detection of binding or hybridization events on a microarray is one of the main challenges in obtaining useful and accurate data. Most of the marketed products are generally made by spotting or ink-jet printing oligonucleotides onto planar, non-porous surfaces such as glass slides. There are sample-labeling kits commercially available that cause the sample oligonucleotide (target) to become labeled with a fluorescent dye. Often it is a fluorescent dye sold under the trademarks of TEXAS RED®, or CY® dyes including CY3 DIRECT® and CY5 DIRECT®. Most commonly, the microarray is "read" through a common fluorometer arrangement with either microscopic magnification or imaging stitching. Reading involves looking for fluorescence at the known locations where the probe was spotted or synthesized. Reading fluorescence of a microarray to detect a binding event is the detection method universally used. However, there are optical issues, difficulty in labeling with fluorescent dyes, occasional high background problems, and most importantly, extremely high costs associated with fluorescent microscopic equipment. Therefore, there is a need to detect binding events on microarrays using lower cost equipment having less complexity while providing reduced variability.

The present inventive method uses electrochemical detection of binding events to provide a lower cost method with less variability. The method involves application of a voltage or current to electrodes on an electrode microarray to detect a binding event. The voltage or current is used to detect a product of an enzymatic reaction or the impact of a product of an enzymatic reaction. The product can be oxidized at an anode or reduced at a cathode. The electrodes on a microarray may function as either anodes or cathodes. The local current or voltage signal is restricted to being detected only at the active electrode and not at neighboring electrodes. The absence of such restriction is referred to as "cross-talk" between electrodes. The present invention provides minimal or no crosstalk between electrodes during detection of a binding event. Preferably, the binding event is detected by a change in the detected voltage or current as compared to an electrode not having a binding event. Alternatively, the binding event can be detected as a change in resistivity. Preferably, the electrode microarray is a CombiMatrix Corporation CUSTOMARRAY12K™ (approximately 12,000 electrodes per square centimeter). Alternatively, the electrode microarray is a CombiMatrix Corporation CUSTOMARRAY902T™ (approximately 1,000 electrodes per square centimeter). Other electrode microarrays are suitable to practice the present invention. Generally, any density of electrodes on a microarray is suitable to practice the present invention provided sufficient isolation of each electrode may be obtained.

### Immunoassays on an Electrode Microarray

Immunoassays are based upon the ability of antibodies to form complexes with only a small number of analytes, which are generally antigens or haptens. Such selectivity of antibodies for specific analytes provides a highly specific assay that is also highly sensitive. In general, immunoassays are based upon the binding of one or more antibodies to analytes. Examples of analytes include antigens, haptens, viruses, bacteria, cells, proteins, polysugars, biological polymer molecules, lipids, glycoproteins (alpha-1-acid glycoprotein,) ricin, M13 phage, Bacillus globigii (BG) spores, fluorescein, rabbit IgG, goat IgG, DNA, RNA, single-stranded DNA, ribosomal RNA, mitochondrial DNA, cellular receptors, glycosylated membrane-bound proteins, non-glycosylated membrane-bound proteins, polypeptides, glycosylated polypeptides, antibodies, cellular antigenic determinants, organic molecules, metal ions, salt anions and cations, and organometallics, and combinations thereof. Problems associated with immunoassays arise from the ability (1) to assemble structures that can be detected and (2) to accurately detect when antibody binding occurs. Ideally, antibody binding occurs only at the locations having the proper probes. The antibody-binding event can be indirectly measured by the presence of a product of an enzymatic reaction.

After the antibody is bound, there needs to be a method of detecting the bound antibody. Generally, the most common methods of detection involve using labels that include radioactive markers, enzymes, or fluorescent markers. The most common method is the use of a fluorescent tag attached to a moiety bound to the antibody or attached directly to the antibody. In this method, a fluorescent imaging system is used to view the locations on a microarray having the antibody. The combination of the fluorescent image and knowing the probe identity provides the assay of the target. The most common immunoassay using an enzyme label is enzyme-linked immunosorbent assay (ELISA.) The most popular enzyme-based immunoassays are the sandwich method and the competitive binding method. Most traditional immunoassays are performed with 96-well microtiter plates; there are other plates available such as 384-well plates and higher. Generally, limitations of conventional immunoassay include the following: (1) the difficulty of multiplexing; (2) the time for analysis is relatively long; (3) the process is multi-stage resulting in complexity and need for adequately trained personnel; (4) practically, the equipment cannot be made smaller; (5) automation is done but is difficult; and (6) it must be done in a laboratory setting. Therefore, there is a need in the art for an improved immunoassay having reduced complexity, analysis time, ability to be miniaturized, automatic, and flexibility to be done in non-laboratory settings.

The present inventive method uses the sandwich immunoassay and an enzyme attached to a reporter antibody. In a sandwich immunoassay on an electrode microarray, first bound antibodies are bound to the microarray at known locations. Generally, the first bound antibodies are referred to as capture antibodies. Preferably, the first bound antibodies are attached to an oligonucleotide that is complementary to oligonucleotides synthesized *in situ* on the electrode microarray at known locations. Alternatively, the oligonucleotides on the electrode microarray are spotted at known locations. The first bound antibodies are attached to the microarray by hybridization to the complementary strands on the microarray thus providing a map of first bound antibody locations according to the location of the complementary strands. Such hybridization and mapping is referred to as a self-assembling microarray.

A solution having analytes is contacted to the microarray to allowing binding of analytes to antibodies. Analytes of interest generally bind only to specific antibodies thus providing high specificity. A solution having a second antibody is contacted to the microarray to allow attachment of the second antibody to the bound analytes. The second antibody is used as a reporter antibody, which means that the antibody reports the microarray locations having analytes attached thereto.

Preferably, the reporter antibody will have an enzyme covalently attached thereto. Alternatively, the reporter antibody may contain a biotin molecule. To this biotin molecule, a streptavidin-enzyme conjugate or an avidin-enzyme conjugate can be attached. Alternatively, a streptavidin may be attached to the biotin followed by another biotin attaching to the streptavidin, wherein the second biotin is covalently attached to an enzyme. Alternatively, an anti-species antibody having an enzyme attached thereto may be attached to the reporter antibody. Alternatively, the reporter antibody may have a streptavidin or avidin bound thereto. A biotin tagged enzyme is then attached to the streptavidin or avidin. Alternatively, the reporter antibody may have an oligonucleotide attached thereto. A complementary oligonucleotide having an enzyme attached thereto is hybridized to the oligonucleotide attached to the report antibody. Preferably, the enzyme is an oxidation-reduction enzyme. Alternatively the enzyme is one causing a cleavage reaction thus producing a redox product, which is a product that is oxidizeable or reducible at the electrode. Alternatively, the product is a solid that deposits on the electrode; the solid product can be detected by resistance, conductivity, or by redox reaction.

The inventive process can be constructed and used with immunoassays, which includes sandwich-type immunoassays. Construction and use provides that enzymes are to be attached to an antibody complex. The complex is formed when a reporter antibody binds to an analyte that is bound to a probe antibody attached at a known location on the microarray. The sandwich assay format allows the use of numerous formats without the difficulty of providing (synthesizing) analyte-based individual antibody-enzyme conjugates. Examples of immunoassays in a sandwich configuration are shown in Figures 1 and 2. Other formats described previously may be used.

### Enzyme Systems for Immunoassays

Different enzyme systems are exemplified herein. Each has a different activation potential. Each function in a unique pH range. Some may require redox mediators. The substrates may vary for each system. The product may be a soluble product that is directly oxidizeable or reducible; the product may be insoluble and deposit on the site having the enzymatic moiety attached thereto. Reactions schemes are provided for each system.

### Horseradish Peroxidase Enzyme

In one embodiment, Horseradish peroxidase (HRP) is used as an enzyme. The enzyme is small (approximately 36 kilodalton) and has a large turnover (maximum initial rate of an enzyme-catalyzed reaction at substrate saturation.) HRP is an oxidation enzyme that catalyses the reduction of hydrogen peroxide, which may damage certain polymeric porous matrices or membranes. Figure 5 shows a reaction scheme catalyzed by HRP using catechol and hydrogen peroxide as the substrates. HPR will catalyze reactions of other substrates with hydrogen peroxide. For example, other substrates include, oxidizeable aromatics, ferrocene derivatives, and oxidizeable inorganic compounds. The HRP catalyzed reaction using catechol and hydrogen peroxide is as follows:

Catechol + H₂O₂ → Quinone + H₂O.

The redox reaction for detection (amperometric detection) of the antibody binding event at the electrode (cathode) having the enzyme complex bound thereto is as follows:

Quinone + 2e⁻ + 2H⁺ → Catechol.

Preferably, the assay is performed at -0.3 volt versus a platinum wire. Preferably, the assay solution is 0.05 molar sodium-citrate-phosphate buffer containing 0.2 molar disodium sulfate at pH 5.0. Preferably, the hydrogen peroxide concentration in solution is 1 millimolar. Preferably, the catechol concentration in solution is 1 millimolar.

Iodine and hydrogen peroxide is another substrate pair that may be used with HRP. The HRP catalyzed reaction using iodine and hydrogen peroxide is as follows:

2I⁻ + 2H⁺ + H₂O₂ → I₂ + 2H₂O.

The redox reaction for amperometric detection of the antibody binding event at the electrode (polarity set as a cathode) having the enzyme complex bound thereto is as follows:

I₂ + 2e⁻ → 2I⁻.

Ortho-phenyldiamine (OPD) and hydrogen peroxide is another substrate pair that will work with HRP. The HRP catalyzed reaction using OPD and hydrogen peroxide is as follows:

OPD+ H₂O₂ → ox-OPD + H₂O.

The redox reaction for amperometric detection of the antibody binding event at the electrode (cathode) having the enzyme complex bound thereto is as follows:

ox-OPD + 2H⁺ + 2e⁻ → OPD.

Preferably, the assay using OPD is performed at -0.1 volts versus a platinum wire. Preferably, the solution is 0.05 molar sodium-citrate-phosphate buffer containing 0.2 molar disodium sulfate and at pH 5.0. Preferably, OPD and hydrogen peroxide are both approximately 1 millimolar in concentration.

### Laccase Enzyme

In another embodiment, laccase is used as an enzyme. Laccase is an oxidation enzyme similar to HRP, but oxygen is substituted for hydrogen peroxide. Laccase will catalyze reactions of other substrates with oxygen. For example, other substrates include ortho-phenylene diamine (OPD) and oxidizeable aromatics. The laccase catalyzed reaction using catechol and oxygen is as follows:

Catechol + O₂ → Quinone + H₂O.

The redox reaction for amperometric detection of the antibody binding event at the electrode (cathode) having the enzyme complex bound thereto is as follows:

Quinone + 2e⁻ + 2H⁺ → Catechol.

Preferably, the assay is performed at -0.3 volt versus a platinum wire. Preferably, the assay solution is 0.05 molar sodium-citrate-phosphate buffer containing 0.2 molar disodium sulfate at pH 5.0. Preferably, the catechol is at 1 millimolar.

### Beta-Galactosidase Enzyme

In another embodiment, beta-galactosidase is used as an enzyme. Beta-Galactosidase catalyzes a reaction that cleaves penultimate beta-galactose residues from oligosaccharides and from glycosyl derivatives. A reaction scheme for beta-galactosidase is shown in Figure 3. Preferably, the substrate is X-Gal (5-bromo-4-chloro-3-indolyl-beta-D-galactopyranoside.) X-Gal is a non-inducing chromogenic substrate for beta-galactosidase. Beta-Galactosidase hydrolyzes X-Gal and forms a blue precipitate. The turnover rate for beta-galactosidase is lower than that for other enzymes, but the enzyme is very stable at neutral pH. Preferably, the X-Gal is approximately 0.1 millimolar in an aqueous solution. Preferably, the X-Gal is dissolved in dimethyl formamide and then added to approximately 0.01 molar phosphate buffered saline while stirring vigorously.

The pH is preferably 7.0. X-Gal does not interact well with glass or metal electrodes. Thus, an electron mediator is needed to shuttle electrons to the electrode surface. Preferably, the electron mediator is a ferri/ferro cyanide solution, which comprises potassium ferrous cyanide and potassium ferric cyanide.) Preferably, the solution is a 50:50 ferric to ferrous. Preferably, the iron concentration from the ferri/ferro cyanide solution is approximately 10 millimolar for amperometric detection. Preferably, the total concentration of iron in solution is adjusted lower. Potentiometric detection is application of a selected potential to an electrode versus a reference electrode while not passing a current through the circuit connecting the electrodes. An example of a reference electrode is silver/silver chloride. The concentration of ferri/ferro cyanide may be increased proportionately with the concentration of the added substrate. Preferably, the voltage settings are 0 Volts (platinum electrode) and 0.5V for the electrode microarray.

### Glucose Oxidase Enzyme

In another embodiment, glucose oxidase is used as an enzyme. Glucose oxidase is an oxidation enzyme. A reaction scheme is shown in Figure 4. Preferably, the reaction takes place at approximately pH 7.5 in a buffer. Preferably, the buffer is phosphate buffered saline having a concentration of approximately 0.01 molar. Preferably, the substrate is glucose, which is highly soluble in water. Preferably, the enzyme is regenerated using 5-methyl-phenazinium methyl sulfate (PMS.) PMS uses the ferro/ferri cyanide shuttle for detection at an electrode. Preferably, the potential at an off-microarray platinum electrode is set to 0 volts, and the microarray electrode potential is set to 0.5 volts.

The glucose oxidase enzymatic reaction has some advantages and drawbacks relative to beta-galactosidase enzymatic reaction. The advantages are that all components of this reaction are extremely soluble in aqueous buffer. However, an electron mediator is required, such as PMS. PMS tends to "air oxidize" over time and must be made up freshly or stored under appropriate inert conditions or it will give a higher background signal.

### Glucose Dehydrogenase Enzyme

In another embodiment, glucose dehydrogenase is used as the enzyme. A reaction scheme for glucose dehydrogenase using substrates glucose and 2,6-dichlorophenolinophenol (DCPI) is as follows:

Glucose + DCPI (ox) → Gluconic Acid + DCPI (red).

The redox reaction for amperometric detection of the antibody binding event at the electrode (anode) having the enzyme complex bound thereto is as follows:

DCPI (red) → DCPI (ox)+ 2e⁻.

Preferably, the assay is performed at 0.3 volt versus a platinum wire. Preferably, the assay solution comprises approximately 50 millimolar piperazine-N,N'-bis(2-ethanesulfonic acid) (PIPES) buffer at pH 6.8 containing approximately 1 nanomolar of pyrroloquinoline quinone (PQQ), approximately 1 micromolar calcium dichloride, approximately 64 micromolar DCIP, and approximately 120 millimolar glucose.

More preferably, electrochemical detection using GDH is performed in PBS solution containing CaCl₂, PQQ, 2,6-Dichlorphenolindophenol (DCIP), phenazine ethosulfate (PES), and glucose. The detection is performed at approximately +300 to 500 millivolts versus a common ITO counter electrode. GDH catalyzes glucose oxidation, and the reaction is accompanied by reduction of PES. The reaction is as follows:

Glucose + PES (oxidized) → Gluconic acid + PES (reduced)

Following this reaction, the reduced PES then reduces DCIP:

DCIP (oxidized) + PES (reduced) → DCIP (reduced) + PES (oxidized)

The reduced form of DCIP is then oxidized by applying a positive voltage to the electrode. The oxidation reaction results in an oxidation current that is detected according to the following equation:

DCIP (reduced) - electron → DCIP (oxidized).

### Alkaline Phosphatase

In another embodiment, alkaline phosphatase (AP) is used as an enzyme. AP catalyzes a de-phosphorylation reaction of aminophenol phosphate (substrate) in the presence of water. The reaction provides for the formation of aminophenol, which is the product that is detected. The reaction scheme is as follows:

Aminophenol phosphate + H₂O →^{(AP)} phosphate + Aminophenol

Aminophenol is detected at an electrode by electrochemical oxidation at the electrode having the AP attached thereto. The current detected is anodic current. The magnitude of the current is proportional to the amount of aminophenol formed. The amount of aminophenol formed is a result of the enzymatic reaction and thus is proportional to the amount of AP bound to the electrode.

### Multiplexed Redox Enzyme Amplified Detection

In another embodiment, each analyte-specific antibody, the target or second antibody attached to the microarray, is tagged with a different and unique enzyme. For each enzyme system, the chemistries are unique so that there is limited (or no) crosstalk during signal acquisition. An example is provided for the use of various enzyme systems. Preferably, at least two different enzymes are used and are selected from the group consisting of horseradish peroxidase (HRP), laccase (Lac), glucose dehydrogenase (GDH), glucose oxidase (GO), and beta-galactosidase (beta-Gal). An example is provided of the use of the first three on a common electrode microarray. However, numerous enzyme systems may be used on the same electrode microarray such that when the substrate solutions (containing buffers and various enzyme substrates) are changed, the specific enzyme produces a signal only under conditions suitable for that specific enzyme and not any other enzyme attached to the microarray.

HRP is a hydrogen peroxide oxidoreductase, which reduces hydrogen peroxide while concomitantly oxidizing a host of organic compounds. Laccase (an oxygen oxidoreductase) oxidizes compounds similar to those acted upon by HRP, but it reduces oxygen to water. Because they have similar enzyme specificities (both are measured as cathodic current), the order of the substrate/buffer addition must be controlled. With the glucose dehydrogenase enzyme system, glucose is oxidized to gluconic acid, while 2,6-Dichlorophenolinophenol (DCPI) is reduced. Anodic current is measured while reduced DCPI is re-oxidized. There is no overlap of the glucose dehydrogenase enzyme system with HRP or laccase because anodic current is measure instead of cathodic current. Additionally, the substrates are substantially different.

When laccase and HRP are used in the same immunoassay system, the choice of which substrate to introduce first is important to perform a proper assay. The combined laccase and HRP system is not affected by the chemistries as written for each enzyme system but rather by the electrochemistry at the electrodes during the detection step. This complication in detection is a result of the production a small trace of hydrogen peroxide at a cathode when organic substrates are reduced. This production of peroxide complicates detection because the substrates for both laccase and HRP enzymes are similar. Hence, running the laccase assay first always gives a trace of a positive reaction for immunoassays system utilizing HRP, which must be taken into account. However, a large quantity of peroxide inhibits laccase chemistries; thus, when attempting to measure enzymatic chemistries using both HRP and laccase, the laccase enzyme assay should be performed first to prevent laccase inhibition by peroxide.

In another embodiment of an enzyme amplification system having more than one enzyme, glucose dehydrogenase (GDH) and HRP are used as the enzymes. Selected electrodes on an electrode microarray have probes synthesized thereon. Other electrodes have random sequences synthesized thereon. Complementary targets are used to self-assemble the GDG and HRP on the microarray. In one embodiment, pyrroloquinoline quinone (PQQ) is used as a cofactor for the GDH system. GDH catalyses glucose oxidation by a reaction involving an electron accepting substrate as follows:

Glucose + electron acceptor → Gluconic acid + reduced electron acceptor (electron donor)

Detection of the electron donor formed as a result of enzymatic reaction can be performed on the electrode as follows:

Electron donor - electron → electron acceptor

In contrast to HRP detection, GDH detection is performed at higher electrode potential (HRP = -0.1 volts, GDH = +0.3 volts), and anodic current is detected in contrast to cathodic current for HRP, i.e., the current measured from the products of the HRP and GDH reactions can be measured separately without interference because the currents are of different polarity; one is positive and one is negative. Therefore, these two enzymes can be used to design dual enzyme system detection, similar to two color optical detection. Preferably, TMB is used as the substrate for HRP in the presence of hydrogen peroxide.

### Redox Enzyme Amplified Detection by Resistance Resulting from an Insoluble Deposition Product

In another embodiment of the present invention, an enzyme substrate reacts and forms a deposit at the microarray electrodes having an enzyme attached thereto. Preferably, the enzyme is HRP. Selected substrates of Horseradish Peroxidase (HRP) will form an insoluble product as a result of enzymatic reaction. Preferably, the substrate is 3,3'-diaminobenzidine (DAB), which forms an insoluble product upon enzymatic oxidation by HRP in the presence of hydrogen peroxide. Other substrates, such as ferrocene derivatives, are suitable to practice the present invention.

Without being bound by theory, as the enzymatic reaction progresses, the oxidized product forms an insoluble compound that is deposited on the surface layer of the microarray. Thus, as the number of enzymes increases at a microarray location, the greater the quantity of insoluble material that is produced and found at the porous reaction layer of sites having the enzymes bound thereto. This layer of insoluble material provides "insulation" on the surface of the electrode. Electrodes having the deposit have altered resistance compared to electrodes not having a deposit. Such altered resistance can be monitored by electrochemical readout of the electrodes. A schematic of two electrodes illustrating the present invention is provided in Figure 20, which shows one electrode having an enzyme and a deposit and another electrode not having an enzyme or a deposit.

Preferably, a current mediator accomplishes the electrochemical readout of the electrodes. Preferably, the mediator is the ferri/ferrocynanide couple, which is sensitive to the amount of the insoluble deposit. Without being bound by theory, the insoluble deposit will reduce the current flux for electrochemical reduction of K₃[Fe(CN)₆] (K₃[Fe(CN)₆]/K₄[Fe(CN)₆] system.) A schematic of two electrodes illustrating a current mediator is shown in Figure 21, which shows one electrode having an enzyme and deposit and another electrode not having an enzyme nor a deposit and thus higher current.

There are numerous advantages of electrochemical detection using a deposition layer as a product of the enzyme reaction. First, the method combines both enzymatic amplification and signal collection principles to improve sensitivity. Thus, theoretical assay detection limit is one molecule per spot. In one embodiment, a detection of 10 femtomolar of 15-mer oligonucleotide was achieved for non-optimized conditions. Second, although one additional stage of the procedure is needed because the enzymatic reaction and the detection procedure are separated, the electrochemical detection does not require fast detection procedure because of the separation. Detection can be performed in tens of minutes or longer without any change in chemistry of an electrode microarray. Finally, since the detected signal is a measure of current reduction caused by the material that has been deposited, high current magnitude (for both anodic or cathodic current) can be applied. Varying the concentration of the redox mediator and/or applied voltage as well as deposition times can be used to control current density. Thus, instrumentation for low current measurement is not required even though relatively low dynamic range may be a possibility. This lack of a need for low current measurement is especially important for high density/low electrode size microarrays where ultra low current measurement may be a limiting parameter.

### Redox Enzyme Amplified Detection by Reduction of Insoluble Deposition Product

In another embodiment of the present invention, a biorecognition-binding event such as DNA hybridization or antibody-antigen interactions are detected using an enzyme tagged target. The enzyme catalyzes deposition of an insoluble species, which is electrochemically active. Detection is accomplished electrochemically by reduction of the insoluble species. As with other embodiments, this embodiment is an alternative to fluorescence detection by the transduction of binding into an electrochemical signal. Transduction is accomplished by using a target tagged with an enzyme that is subsequently exposed to a substrate and forms a reaction product that is a surface bound electro-active molecule, cluster of molecules, or polymerized molecules. The electro-active product can be detected by applying a voltage or current to the electrode having the probe-target-enzyme complex. This scheme alleviates the requirement of costly optics in fluorescence techniques and has the added benefit of enzymatic amplification of the signal by the initial binding event.

The current embodiment of the present invention provides a method of measuring binding events that ameliorates the problems associated with fluorescence. In the current embodiment, an enzyme-amplified approach is provided, whereby the enzyme product is insoluble and deposits on the electrode surface having the probe-target-enzyme complex. The insoluble enzyme product is electrochemically active and can be converted into an electronic signal by applying a voltage to the surface and monitoring the resulting current. The enzyme product is reduced during monitoring.

In the preferred embodiment, the enzyme is HRP and the substrates are hydrogen peroxide and tetramethybenzidine (TMB). HRP oxidizes TMB in the presence if hydrogen peroxide thus forming an insoluble oxidized molecule or cluster of molecules that deposits on the electrode having the HRP bound thereto. TMB is removed and replaced by a buffer to stop the oxidation reaction. The oxidized TMB is detected by applying a voltage to the electrode to reduce the oxidized enzymatic product. Other enzyme substrate combinations that are capable of forming insoluble reactions products are suitable to practice the present embodiment with the caveat of the insoluble reaction product being capable of being reduced or oxidized during detection.

In a preferred embodiment, the surface concentration of oxidized TMB is proportional to the amount of enzyme at the site, the concentration of both peroxide and TMB in solution, and the total reaction time. This general relationship holds for any combination of enzyme and substrates. All of the variables are controllable using this method. Once the insoluble precipitate is bound to an electrode surface, the application of a reducing potential will change the oxidation state and yield faradaic current proportional to the amount of oxidized species on that electrode. The detection of this current is the basis for quantifying the amount of binding, such as RNA-DNA on the electrode surface.

The detection scheme illustrated here is similar to a stopped flow experiment, wherein enzyme reaction conditions, such as time and substrate concentration, can be controlled. The resulting electrical signal is directly proportional to the amount of enzyme product on the surface and is a function of substrate concentration and reaction time. Since both of these variables can be controlled using this method, quantification can be performed.

### Redox Enzyme Amplified Detection by Sequential Reading

In another embodiment of the present invention, a method is provided for reading an electrode microarray having enzyme product detectable by a redox reaction. To read an electrode microarray, all electrodes are set to a low potential that is slightly positive of ground. Preferably, the potential is approximately 10 millivolts. This low voltage state is maintained while all electrodes are at open circuit by not providing any path to a low resistance ground. By imposing an open circuit, no current can flow. The lack of current prevents any change in the redox state of a product. Each electrode is sequentially set to ground and then returned to the low potential with an open circuit. No two electrodes are set to ground and then returned to low potential with an open circuit at the same time.

When an electrode is set to ground, the electrical circuit is completed such that current will flow from the electrode towards the remaining part of the electrode microarray because of the low positive potential. This current flow will reduce the enzyme product on the individual electrode and oxidize material at other electrodes on the microarray. This sequential scheme effectively uses the entire microarray as a counter electrode and the grounded electrode as a working electrode. Preferably, current is measured at the grounded electrode immediately after the electrode is set to ground. To read the entire microarray, each individual electrode is sequentially switched to ground, read, and switched back to the applied potential. The measured current is a linear combination of both charging and faradaic current. Charging current is minimized due to the low overpotential, e.g., 10 millivolts and is overshadowed by the faradaic current. Alternatively, a negative potential of approximately -10 millivolts may be used for reading a microarray by oxidizing a product at electrodes.

The enzyme reaction can occur for a set amount of time and then be immediately stopped at a predetermined end point. At this stage, the dynamic process is stopped; therefore, there is no time constraint on the detection of the enzymatic reaction. Electrode microarrays have been prepared according to the present invention where the enzyme reaction was stopped and then followed by reading of the microarray 18 hours later. Very low overpotentials are used; therefore background current due to charging is at a minimum. This minimum background current will enhance signal to noise ratios. Only one electrode is used as a working electrode. The remainder of the microarray is effectively a counter electrode; therefore, there is no need to use a separate "off microarray" electrode to serve this purpose. Additionally, since all electrodes are relegated to serve as a counter electrode at some point, all electrodes are available for assay purposes.

### Example 1: Horseradish Peroxidase

In a first immunoassay, a product, quinone, of the HRP catalyzed reaction was detected amperometrically. This assay was performed at -0.3 V versus a platinum wire in an aqueous solution having 0.05 molar sodium-citrate-phosphate buffer at pH 5.0 while containing 0.2 molar disodium sulfate. The concentration of catechol was 1 millimolar, and the concentration of hydrogen peroxide was 1 millimolar. To attach HRP, probe DNA was synthesized *in situ* in a checkerboard pattern on the electrode microarray. Standard phosphoramidite chemistry was used with electrochemically-generated acid to remove the protecting groups. The target DNA, which was complementary to the probe DNA, was tagged with a biotin. The target DNA was hybridized to the probe DNA at 40 degrees Celsius for one hour using a solution of one nanomolar target DNA in 2XPBST.

HRP having a streptavidin tag was added to attach to the biotin using a solution of the HRP-streptavidin complex. The complex comprised a proprietary polymer of 80 HRP units conjugated to streptavidin. The streptavidin-HRP complex was purchased from Research Diagnostics, Inc. To make the streptavidin-HRP solution, one microliter of the streptavidin-HRP 80 complex was diluted to 5000 microliters using 2XPBST. The time of exposure was 60 minutes and the solution temperature was 25 degrees Celsius. The results show that the expected checkerboard pattern from the electrode microarray was obtained as evidenced by increased current flow at the electrodes having the enzyme attached thereto, i.e., the locations having binding of HPR were detected by having a higher cathodic current than those locations not having HRP. The higher current is indicative of the reduction of quinone to catechol at the electrode. Additionally, there was binding at the expected locations without the presence of noise or crosstalk between neighboring electrode locations.

### Example 2: Laccase

In a second immunoassay, a product, quinone, of the laccase-catalyzed reaction was detected amperometrically. This assay was performed at -0.3 V versus a platinum wire in an aqueous solution having 0.05 molar sodium-citrate-phosphate buffer at pH 5.0 while containing 0.2 molar disodium sulfate. The concentration of catechol was 1 millimolar, and the concentration of oxygen was not measured but consisted of the amount naturally present in solution. To attach laccase, target DNA was synthesized *in situ* in a checkerboard pattern on the electrode microarray. Standard phosphoramidite chemistry was used with electrochemically-generated acid to remove the protecting groups. The target DNA, which was complementary to the probe DNA, was tagged with a biotin. The target DNA was hybridized to the probe DNA at 40 degrees Celsius for one hour using a solution of one nanomolar target DNA in 2XPBST. Streptavidin was added to attach to the biotin using a solution of 1 microgram per milliliter streptavidin in 2XPBST, a temperature of 25 degrees Celsius, and a time of 60 minutes. Laccase having a biotin tag was added to attach to the streptavidin using a solution of 1 microgram per milliliter laccase-biotin complex in 2XPBST, a temperature of 25 degrees Celsius, and a time of 60 minutes.

The results showed that the expected checkerboard pattern from the electrode microarray was obtained as evidenced by the increased current at the electrodes having the laccase bound thereto, i.e., the locations having binding of laccase were detected by having a higher cathodic current than those locations not having laccase. The higher current is indicative of the reduction of quinone to catechol at the electrode. Additionally, there was binding at the expected locations without the presence of noise or crosstalk between neighboring electrode locations.

### Example 3: Beta-Galactosidase Immunoassay

In an immunoassay on an electrode microarray, beta-galactosidase was used as the enzyme, and the substrate was X-Gal. Biotinylated beta-galactosidase was used. The reactions were carried out at pH 7.0 in phosphate-buffered saline. The concentration of the buffer was 0.01 M. To make a solution, a selected quantity of X-Gal was dissolved in DMF because it is very soluble in DMF and has low solubility in water. The DMF with X-Gal was added to the aqueous buffer. The final concentration of X-Gal was approximately 0.1 millimolar, which is near the saturation concentration. During addition of the DMF with X-Gal to the buffer, the buffer was vigorously stirred to prevent precipitation of the X-Gal because of the limited solubility of the X-Gal water. If the DMF with X-Gal solution is added without vigorous stirring, the X-Gal will precipitate. The instability of this solution requires that a fresh solution be prepared daily. A ferri/ferro cyanide solution was used at about a 10 millimolar concentration for amperometric detection. The voltage settings were 0 volts (off-microarray platinum electrode) and 0.5 volts for the electrode microarray. Selected electrodes on an electrode microarray (CombiMatrix Corporation) had probe DNA synthesized *in situ* by electrochemical synthesis (standard phosphoramidite chemistry with electrochemically-generated acid to remove protecting groups.) The last DNA unit attached to the probe DNA was modified to contain a biotin tag. Streptavidin was added to the microarray to bind to the biotin. Redox reagents and substrates were added according to the reaction scheme in Figure 3.

Results for beta-galactosidase immunoassay detection system are shown in Figure 6. In one section of the microarray represented by lanes 1-8, no biotin tag was placed. In lanes 9-16, biotinylated beta-galactosidase was added to the microarray to bind to the streptavidin thus forming a biotin-streptavidin-biotin complex. These data show an amperometric response difference in the two parts of the microarray, that is, lanes 1-8 versus lanes 9-16 containing beta-galactosidase. The negative current values of selected electrodes shown in Figure 6 indicate that beta-galactosidase is bound thereto. The neighboring electrodes were used as counter electrodes in the synthesis process and did not contain biotin tags. Similarly, Figure 7 shows this microarray profile as a slice (row) of the three-dimensional plot shown in Figure 6. Electrodes 9, 11, 13, and 15 contain beta-galactosidase, and as expected, the current on those electrodes is higher in absolute value.

### Example 4: Beta-Galactosidase Immunoassay using Fluorescein

In an immunochemical detection experiment, a capture and detection of fluorescein-containing beta-galactosidase was performed. Probe DNA was synthesized *in situ* on an electrode microarray (CombiMatrix Corporation) using standard phosphoramidite chemistry and electrochemically-generated acid for deblocking of the protecting groups on the phosphoramidites. The target DNA was complementary to the probe DNA and had an anti-fluorescein antibody attached thereto. The target DNA having the antibody was hybridized to the probe DNA using a solution of approximately 85 micromolar (approximately 2.5 milligrams of target DNA per 200 microliters of buffer), a temperature of 40 degrees Celsius, and a time of 60 minutes. A solution of beta-galactosidase tagged fluorescein was contacted to the microarray to allow the fluorescein to bind to the antibody on the target DNA. The solution concentration was approximately one nanomolar; the temperature was 25 degrees Celsius; and the time of contact was 60 minutes. The fluorescein acted as a low molecular weight antigen attached to the larger enzyme.

The substrate used was X-Gal at 0.1 millimolar in an aqueous solution. To make the substrate solution, X-Gal was dissolved in dimethyl formamide and then added to approximately 0.01 molar phosphate buffered saline while stirring vigorously. The pH was 7.0. A ferri/ferro cyanide solution was used as an electron mediator. The cyanide solution comprised potassium ferrous cyanide and potassium ferric cyanide in a 50:50 ferric to ferrous proportion. The iron concentration from the ferri/ferro cyanide solution was 10 millimolar. The voltage settings were 0 volts (platinum electrode) and 0.5 volts for the electrode microarray.

The results of the amperometric measurements are shown in Figure 8. The probe DNA was located in row two (S2), lanes 1, 3, and 5 of the electrode microarray. The electrode sites containing the fluorescein-beta-galactosidase showed an enhanced value of current according to the reaction scheme of Figure 3. The binding of F-beta-galactosidase to the porous reaction layer covering the electrodes was corroborated and confirmed using an epi-fluorescent microscope. The epi-fluorescent data exactly track the results obtained with the inventive process of detecting current flow at the electrodes.

### Example 5: Glucose-Oxidase Electrochemical Detection

In this example an assay was performed using glucose oxidase having a biotin attached thereto. Probe DNA was synthesized *in situ* on an electrode microarray (CombiMatrix Corporation) using standard phosphoramidite chemistry and electrochemically-generated acid for deblocking of the protecting groups on the phosphoramidites. The target DNA was complementary to the probe DNA and had a biotin tag attached thereto. The target DNA was hybridized to the probe DNA using a solution of approximately 15 nanomolar (approximately 2.5 nanograms of target DNA per 200 microliters of buffer), a temperature of 40 degrees Celsius, and a time of 60 minutes. A solution of streptavidin was contacted to the microarray to attach the streptavidin to the biotin on the target DNA. The solution concentration was 10 nanomolar of streptavidin in 2XPBST; the temperature was 25 degrees Celsius; and the time was 60 minutes.

The enzyme having the biotin tag was attached to the streptavidin using a solution having a concentration of 10 nanomolar enzyme, a temperature of 25 degrees Celsius, and a time of 60 minutes. To detect the enzyme, a substrate solution was used that comprised 0.01 molar buffer at pH 7.5, 40 millimolar beta-D-glucose, 0.3 millimolar PMS, and ferro/ferri cyanide in a 50:50 proportion. The total iron concentration was 10 millimolar. The ferro/ferri cyanide was added as potassium ferrous cyanide and potassium ferric cyanide. Figure 9 shows a slice of a three-dimensional plot of current at selected electrodes. The glucose oxidase is located in lanes 2, 4, 6, and 8. As shown in the figure, current increased in absolute value according to the reaction scheme in Figure 4 on those electrodes having the glucose oxidase bound thereto.

### Example 6: Horse Radish Peroxidase Detection using Catechol

In this example, HRP is the enzyme and catechol and hydrogen peroxide are the substrates. The redox curve for the peroxidase reaction was monitored by a one 100-micrometer diameter electrode on a bare *(i.e.,* no porous membrane or probes synthesized thereon) electrode-containing microarray device (CombiMatrix Corporation). The microarray was immersed in solution, and the electrodes thereon were used to monitor solution redox chemistry. The results are shown in Figure 10. Each data point in Figure 10 represents measurements at the same electrode for each curve. The results indicate that at a negative potential, the amperometric difference with and without HRP enzyme was substantial. The pattern of the results is somewhat like a cyclic voltamogram. An amperometric experiment was performed over a period of time. The results are shown in Figure 11. As shown in Figure 11, the current flow leveled off after a period of time. Thus, the best time to begin taking measurements is after approximately three seconds, which is where the leveling began. Each data point in Figure 11 is a measurement at a different electrode.

### Example 7: HRP Immunoassay of Five Analytes

In this example, electrochemical detection of HRP products was performed on an electrode microarray. Five analytes were detected and include alpha-1-acid glycoprotein (AGP), the plant lectin ricin (RCA), M 13 phage, Bacillus globigii (BG) spores, and fluorescein. The microarray was a CombiMatrix 98001 CMOS chip having 1170 electrodes per square centimeter. The electrodes were approximately 90 micrometers in diameter.

Polyclonal anti-human alpha-1-acid glycoprotein (AGP) from rabbit, monoclonal anti-FITC antibody, human AGP, polyclonal anti-ricin (RCA) antibody (in rabbit serum), RCA, and agarose beads containing covalently attached ricin were obtained from Sigma Chemical Company (St. Louis, MO). The monoclonal anti-phage (M13) and the HRP conjugate of a monoclonal anti-phage antibody were purchased from Amersham Pharmacia Biotech, Piscataway, New Jersey. M13 phage (starter package) was a product from Promega ,Madison, Wisconsin. Additionally, SBCCOM (Aberdeen Proving Grounds) provided the B. globigii spores as well as the polyclonal anti-BG spore antibody (from goat) for this example.

Succinimidyl 4-[N-maleimidomethyl]cyclohexane-1-carboxylate (SMCC) cross-linking reagent, biotin-LC-LCNHS, and activated agarose beads were obtained from Pierce Chemical Company, Rockford, Illinois. P-6 and P-30 spin columns were obtained from Biorad, Richmond, California. The oligonucleotides used to modify the proteins were prepared by Operon (Qiagen, Alameda, CA). The 3' FITC tagged 15-mer complement used as a fluorescein analog was also obtained from Operon.

The polyclonal anti-AGP antibody was affinity purified using an agarose-AGP conjugate column prepared from a kit purchased from Pierce Chemical Company (Milwaukee, WI). The antibody was loaded onto the labeled column in PBS, pH 7.5, and eluted with glycine buffer, pH 2.8. The eluted solution was monitored at A280, and the appropriate fractions were collected in a microtiter plate and neutralized with 10× PBS, pH 7.4; the sample was then dialyzed overnight against 1×PBS, pH 7.4. In a similar fashion, agarose conjugated with ricin was used to affinity purify the rabbit anti-ricin antibody.

Biotinylation of antibodies was accomplished by the reaction of biotin-LC-LC-NHS (1 mg/ml in dimethyl formamide) with the protein in aqueous phase pH 7.4. The molar ratio of biotin to antibody in the labeling reaction was 10:1 (the final concentration of biotin was 70 micromolar). The reaction was allowed to proceed for one hour and then the solution was passed through a Biorad P-6 spin column. The eluate was collected and stored at 4 degrees Celsius for future use.

Antibodies were tagged with oligonucleotides that had complementary sequences to probe oligonucleotides synthesized directly on the microarray, using the following procedure: (1) the oligonucleotide containing a protected thiol group was first reduced with Cleland's reagent and separated on a Biorad P-6 spin column; (2) antibody was then separately reacted with SMCC, labeling reactive amines on the protein and then purified using a P-6 column; (3) the SMCC tagged antibody was then mixed with the thiol containing oligonucleotide so that the oligonucleotide became covalently attached to the antibody; and (4) excess oligonucleotide was removed by using a Biorad P-30 spin column.

The antibodies were allowed to self-assemble on the chip surface using the following procedure. The samples of antibody-oligonucleotide in 2× PBST (phosphate buffer containing 0.05% TWEEN® 20) were incubated with the chip at 40 °C for 1 hour. The solution contained five different antibody-oligonucleotide conjugates (different antibody specificities). Each antibody-oligonucleotide conjugate was self-assembled on the predetermined electrodes, which contained the synthesized oligonucleotide complement. In other words, each antibody had a unique oligonucleotide attached thereto so that the location of the antibody on the microarray was determined by the location of the complementary probe DNA. The probe DNA was synthesized *in situ* on the microarray using standard phosphoramidite chemistry and electrochemically-generated acid to remove protecting groups. The microarray was washed twice with 2× PBST buffer and then allowed to dry after hybridization to the probe DNA. A solution having the analytes was incubated with the activated chip for 1 h. The microarray was washed with 2× PBST buffer and then immersed for one hour in a solution containing the biotinylated anti-analyte antibody (or in the case of phage, the HRP-Ab conjugate). The microarray was washed with 2× PBST and then placed into a solution containing streptavidin-HRP conjugate (0.5 h; for the AGP and ricin analytes only). Finally, the microarray was washed with 2× PBST and kept in 2x PBST until measurements were initiated. Negative controls were determined from a sub-area modified with a synthesized kras sequence (oncogene 15-mer sequence). This sub-area was treated like all the other electrodes (exposed to analytes, biotinylated antibody, SA-HRP and enzyme substrate). The current was measured at each electrode and was deemed to have enzyme bound thereto if the mean signal was three standard deviations above the background.

When BG spores were the analyte, the protocol for capture of these spores by the bound antibody was altered from the procedure detailed above. The spores themselves tended to settle out in the aqueous solution in which they had been suspended. This may be due to aggregation or other factors. The suspension containing the spores was added to the microarray surface and allowed to settle out, although the solution was mixed at times. The spores that were not bound to the selected electrodes via the specific antibody were then washed away in the subsequent wash step.

The enzyme buffer conditions and substrate for the HRP catalyzed reactions were as follows: the potential at the counter electrode was set to 0.3 volts, and the potential on the chip electrode was set to 0 volts. Thus, electrons flowed from the chip to the reaction media as the enzymatic product was converted from substrate by HRP. The reaction buffer contained citrate/phosphate (50 millimolar ) with 0.2M NaCl at pH 5.0. The solution contained 0.003% hydrogen peroxide and 1 millimolar in ortho-phenylenediamine (OPD).

As determined by statistics and maximum signal, 60 minutes is the optimum incubation time for detection of a low concentration of an analyte. However, a shorter incubation time (12 minutes or less) can be used to obtain results more quickly when an analyte is present at higher concentrations. Figure 34 shows the assay response as a function of analyte concentration and incubation time for analytes ricin in (A) and for M13 phage in (B). Incubation times of 12 minutes (curve 1), 30 minutes (curve 2), and 60 minutes (curve 3) were used.

Figure 12 shows the current output from a multiplexed immunoassay for the five analytes.

Analyte incubation was 1 hour. Each analyte-specific antibody was in a 4 × 10 array in a checkerboard pattern (alternate electrodes used). The analyte concentrations were as follows: RCA (1 ng/ml), AGP (2.75 microgram/ml), BG spores (2 × 105 spores/ml), and phage (1010 pfu/ml). For the detection of FITC analyte, 1 microgram of FITC oligonucleotide (complement to sequence on the chip) was incubated on the chip. The chip was next incubated with a biotinylated anti-FITC antibody. This antibody was present in a mixture containing all biotinylated antibodies for every analyte present. The intensities are not the same for all analytes because different concentrations were used. The negative controls were a 15-mer kras sequence, and those are shown in rows 25-27. Additional (kras-modified) controlelectrodes were also located in each row between analyte-specific areas (rows 5, 10, 15, 20). The assay performance for each analyte will differ based upon the affinity constant of each antibody-analyte pair.

### Example 8: HRP Immunoassays

In this example, horseradish peroxidase was the enzyme and catechol and hydrogen peroxide were the substrates. Rabbit IgG and goat IgG were affinity tagged and bound to the upper section and lower section of an electrode microarray, respectively. Binding was accomplished by addition of HRP-tagged anti-goat antibody and anti-rabbit antibody. Detection was made possible using HRP-tagged goat anti-rabbit antibody and HRP-tagged mouse monoclonal anti-goat antibodies. The results of these experiments are shown in Figure 13. Specifically, Figure 13 shows amperometric detection of rabbit IgG bound to selected electrodes on the electrode microarray. The microarray was an electrode microarray obtained from Combimatrix Corporation. HRP-tagged goat anti-Rb was used for the electrochemical detection. Samples are in S4 (1, 3, 5) and S5 (2, 4). The sign of the current flow has been changed for presentation of these data. Further, Figure 13 shows detection of goat IgG bound to selected electrodes. HRP-tagged anti-goat antibody and anti-rabbit antibody was used for the electrochemical detection. Samples are in S1 (1, 3, 5) and S2 (2, 4, 6). These data are in agreement as to what would be expected based upon earlier work using fluorescent-tagged antibodies and standard fluorescence detection.

### Example 9: HRP and OPD Assay of two Targets

In this example, the target oligonucleotides (two) were 15-mer single stranded DNA units with a biotin tag attached to the 3' end and were purchased from Operon. The first target configuration5'-15-mer A-BIOTIN 3', and the second target had configuration 5'-15-mer B-BIOTIN-3'. Probe oligonucleotides complementary to the target oligonucleotides were synthesized *in situ* on a pin grid microarray (PGA) version of an electrode microarray having approximately 1000 electrodes available from CombiMatrix Corporation. The probes were synthesized in a checkerboard pattern on two different areas of the electrode microarray. After synthesis of the probe oligonucleotides, the target oligonucleotides were hybridized to the complementary probe oligonucleotides on the PGA. Hybridization conditions comprised 40 degrees Celsius for 1 hour at a concentration of 1nM of oligonucleotide targets.

The hybridization solution comprised oligonucleotides in 2X phosphate buffered saline 0.05% TWEEN® (PBST). The PGA was washed using 2X PBST. A solution of streptavidin having a horseradish peroxidase tag was incubated with the hybridized PGA microarray. Incubation allowed the streptavidin-HRP complex to bind to the biotinylated hybridized target probe. The incubation time was 45 minutes and the temperature was 25 degrees Celsius. The complex comprised a proprietary polymer of 80 HRP units conjugated to streptavidin. The streptavidin-HRP complex was purchased from Research Diagnostics, Inc. To make the streptavidin-HRP solution, one microliter of the streptavidin-HRP 80 complex was diluted to 5000 microliters using 2XPBST. The PGA was washed using 2X PBST. A substrate solution having 1 millimolar of o-phenylamine diamine (OPD) and 1 millimolar of hydrogen peroxide was contacted to the PGA. A potential of -0.3 volts was applied to the working electrode relative to the counter electrode, and the current was measured. As shown in Figure 14, the electrodes having HRP bound thereto had a higher current due to the reduction of the oxidized OPD product. The higher current is depicted by the three dimensional bar chart and by the dark squares in the two-dimensional insert in the upper right corner.

### Example 10: Potentiometric Detection

Figure 15 shows a three-dimensional plot for oligonucleotide hybridization electrochemical detection. Specifically, Rab and Kras oligonucleotide sequences were *in situ* synthesized on an electrode microarray (CombiMatrix.) The Kras sequence had configuration 5'-15-mer C-3'. The Rab sequence used was 5'-15-mer D-3'. The probe oligonucleotides synthesized by *in situ* electrochemistry techniques had a sequence of 5' 15-mer C'- 3' for Kras locations and a sequence of 5'-15-mer D'-3' for Rab locations, wherein the 15-mer C' was complementary to the 15-mer C sequence, and the 15-mer D' was complementary to the 15-mer D sequence.

A platinum wire counter electrode was used. The target DNA was a biotinylated Rab sample purchased from Operon Biotechnologies, Inc. The biotinylated Rab target was added to the microarray to allow hybridization (1 nanomolar, 40 degrees Celsius, 1 hour) to the probe DNA on the microarray. Streptavidin conjugated HRP was added to the microarray to complex with the biotinylated Rab sequence. Electrode potential versus Ag/AgCl reference electrode was measured at each electrode. These data are shown in Figure 15 as a three-dimensional plot. Positive signal was found for Rab probe oligonucleotide. As expected, no signal was found for Kras probes.

### Example 11: Calibration Plots

In this example, three analytes were mixed into one solution and added to a prepared electrode microarray. The ability of microarray devices to have synthesized many different probe oligonucleotides at different known locations allows for multiple analyte detection on a single chip. In each case, the sample to be investigated was labeled with an enzyme using standard conjugation. Multiple samples were pooled so that all of the targets to be investigated were from a single pooled sample. Samples of AGP, ricin, and rabbit Rab oligonucleotide samples were pooled and investigated on a single microarray (CombiMatrix) using HRP as the enzyme. Only those locations having the appropriate probe detected target even though the group of targets were either protein or nucleic acids. Based upon multiple microarray investigations, the limits of detection were found to be 5 picogram per milliliter for AGP and 300 picogram per milliliter for ricin; this concentration translates to 2.5 femtomolar. The dynamic detection range spanned four logs. The relationship of current to analyte concentration is shown for AGP and ricin in Figure 16.

Figure 17 provides a plot of the negative current versus the log of the concentration of Rab-biotin oligonucleotide target for a hybridization time of 2 hours at 40 degrees Celsius. Each data point in Figure 17 represents the average negative current of a different electrode microarray, wherein each microarray has been exposed to different concentrations of oligonucleotide target.

### Example 12: Multiple Enzyme Immunoassay using HRP and Laccase

In this example, laccase and HRP were used on the same electrode microarray to perform an immunoassay of a mixture of AGP and ricin. As a first step, reagents were prepared for the assay. A 15-mer oligonucleotide was attached to the first antibody via primary amines on the antibody. The antibody was anti-AGP for laccase and was anti-RCA for HRP. The oligonucleotide was modified on the 3' end with a disulfide and had an 18-unit PEG on the 5' end. Thus, the oligonucleotide had a structure as follows: PEG(18)-15-mer DNA-S-S-R. The R group was a methyl but can be another group. Two different 15-mer oligonucleotides were used; one was used for laccase and one for HRP.

To prepare a thio-modifed oligonucleotide, the oligonucleotide having the disulfide (10 microliters of 1 millimolar stock solution) was mixed with 30 microliters of 10xPBS and 5 microliters of dithiothreitol (DTT) solution (1 milligram per milliliter in water). The mixture was then incubated at ambient temperature for 4 hours and applied twice to P6 columns pre-equilibrated with 1X phosphate buffered saline (PBS). The resulting thio-modified oligonucleotide had the following structure: PEG(18)-15-mer-S-H.

To prepare the antibody, a crosslinker was added. Succinimidyl-4-(N-maleimidomethyl) cyclohexana-1-carboxylate (SMCC) was dissolved in dimethyl formamide (DMF) to obtain 1 milligram per milliliter concentration. SMCC solution (1.2 microliters) was added to 50 microliters of the desired antibody solution (Imilligram per milliliter) in 1xPBS. The mixture was then incubated for 2 hours at ambient temperature and applied twice to P6 columns pre-equilibrated with 1xPBS.

The modified oligonucleotide was attached to the modified antibody as follows. The modified oligonucleotide and antibody solutions were combined and incubated for 2 hours at ambient temperature. The mixture was then applied twice to P30 columns pre-equilibrated with 1xPBS. The resulting structure was as follows: 15-mer-PEG(18)-antibody. The oligonucleotide-linked antibody was then collected, frozen, and stored.

An electrode microarray (CombiMatrix) having approximately 1000 electrodes was prepared by synthesizing complementary oligonucleotides on the electrodes. Two different types of complementary oligonucleotides were synthesized (standard phosphoramidite chemistry and electrochemical deblocking) and corresponded to the one used for laccase and the one used for HRP. The locations of each complementary oligonucleotide were controlled.

The first antibodies having the oligonucleotides attached thereto were contacted to the microarray for self-assembling. Both antibodies tagged to the selected oligonucleotide were suspended in 2x PBS containing 0.05% TWEEN® 20 (2x PBST) at approximate concentration of 1-5 micrograms per milliliter. The electrode microarray was incubated with the oligonucleotide-tagged antibodies for 1 hour at 40 degrees Celsius. The microarray was then washed three times with 0.5 milliliters of 2x PBST.

The analytes, ricin for HRP and AGP for laccase, were contacted to the microarray for attachment to the respective antibodies. The analytes of different concentrations were suspended in 2x PBST. The analyte-containing solution (30 microliters) was applied to the microarray. The microarray was incubated with the analyte(s) containing solution for 1 hour at ambient temperature. The microarray was then washed 3 times with 0.5 milliliter of 2xPBST.

The second antibody was then attached to the microarray to form a sandwich. Each second antibody for laccase and for HRP was attached in separate stages, i.e., separate solutions for each antibody. For the HRP stage, biotinylated secondary antibody (0.2 milligrams per milliliter) in 2x PBST was contacted to the microarray. The microarray was incubated for 1 hour at ambient temperature. The microarray was then washed 3 times with 0.5 milliliter of 2x PBST.

An HRP-streptavidin conjugate (1 micrograms per milliliter) in 2x PBST was then introduced to the microarray. The microarray was incubated with the HRP-streptavidin conjugate solution for 1 hour at ambient temperature. The microarray was then washed 3 times with 0.5 milliliter of 2x PBST. Any remaining sites on the streptavidin were blocked by addition of free biotin followed by washing.

For the laccase stage, biotinylated secondary antibody (0.2 milligrams per milliliter) in 2xPBST was contacted to the microarray. The microarray was incubated for 1 hour at ambient temperature. The microarray was then washed 3 times with 0.5 milliliter of 2x PBST. Streptavidin was then contacted to the microarray to bind to the biotin on the secondary antibody for the laccase sandwich. Biotinylated laccase was contacted to the microarray to bind to the streptavidin attached to the biotin on the secondary antibody of the laccase sandwich. Electrochemical detection of the laccase sandwich was performed first followed by electrochemical detection of the HRP sandwich.

In the presence of catechol and atmospheric oxygen, the immunoassays for AGP, using a laccase tagged antibody, gives a positive signal as shown in Figure 18.The assay solution was 0.05 molar sodium-citrate-phosphate buffer containing 0.2 molar disodium sulfate at pH 5.0. The hydrogen peroxide concentration in solution was 1 millimolar. The catechol concentration in solution was 1 millimolar. The temperature was ambient temperature. The applied voltage was -0.3 volts.

As expected, the immunoassay for ricin using an HRP tag shows no signal as shown on the right hand side of Figure 18. When the microarray was washed and placed into a substrate solution containing OPD and hydrogen peroxide, a positive signal was observed for ricin using an HRP tag as shown on the right hand side of Figure 19.The assay using OPD was performed at -0.1 volts versus a platinum wire. The solution was 0.05 molar sodium-citrate-phosphate buffer containing 0.2 molar disodium sulfate and at pH 5.0. OPD and hydrogen peroxide were both 1 millimolar in concentration. The temperature was ambient temperature. A low intensity response can also be observed for the laccase-tagged antibody associated with the detection of AGP.

The order of the assay must be followed as outlined in this example when using the sandwich as defined in this example. In other words, the laccase detection must be before the HRP detection. Otherwise, enzyme cross talk may occur. The cross talk will be evident because the enzymes are from related families and thus oxidize the same organic compounds by either reducing oxygen or hydrogen peroxide. A multiplexed enzyme system may become more robust by the use of two dissimilar redox enzymes. One of many other enzyme systems that could be used is glucose dehydrogenase with either laccase or HRP.

### Example 13: Multiple Enzyme Immunoassay using HRP and Glucose dehydrogenase

In this example, HRP and glucose dehydrogenase (GDH) were used on the same electrode microarray to demonstrate the feasibility of using these enzymes in a multiple enzyme immunoassay. The microarray was a CombiMatrix CUSTOMARRAY 12K™. GDH and HRP were detected separately and sequentially on the microarray. GDH and HRP were attached to the microarray using hybridization of oligonucleotides to probes synthesized on the microarray. Before attaching both GDH and HRP to the same microarray, GDH was attached to a microarray by itself to demonstrate the use of GDH. Attachment was accomplished using hybridization of three selected biotinylated oligonucleotides.

Several steps were used to prepare a microarray having GDH. GDH was biotinylated according to standard Pierce biotinylation protocol. To attached GDH to the microarray, three probes complementary to three different biotinylated oligonucleotides were synthesized *in situ* on a microarray using electrochemical synthesis. The probes comprised aP1 having sequence 15-mer E', aP2 having sequence 15-mer F', and aP3 having sequence 15-mer G'. The targets were 15-mer in length and complementary to the probes. Electrodes not having the probes had random 15-mer sequences synthesized thereon. The biotinylated oligonucleotides were hybridized to the respective complementary probes by contacting the microarray to a solution of the oligonucleotides, which were designated as P1, P5, and P6. The biotin was attached at the 3' end. The concentration of P1, P5, and P6 in solution was 1 nanomolar, 50 picomolar, and 10 picomolar respectively. The hybridization time was 60 minutes at a temperature of 40 degrees Celsius. A solution having streptavidin at a concentration of 1 microgram per milliliter was contacted to the microarray to attach streptavidin to the biotin on each oligonucleotide. Biotinylated GDH in PBS containing 1 millimolar CaCl₂ and 10 micromolar PQQ was then incubated at 25 degrees Celsius for 15 minutes with the microarray to form a complex having the following structure: hybridized oligonucleotide-biotin-streptavidin-biotin-GDH.

Electrochemical detection of the GDH was performed in PBS solution containing 100 micromolar CaCl₂, 0.1 micromolar PQQ, 1 millimolar 2,6-Dichlorphenolindophenol (DCIP), 600 micromolar phenazine ethosulfate (PES), and 100 micromolar of glucose. The detection was performed at +300 millivolts and then at +500 millivolts versus a common ITO counter electrode. GDH catalyzes glucose oxidation, and the reaction is accompanied by reduction of PES. The reaction is as follows:

Glucose + PES (oxidized) 〉̶ Gluconic acid + PES (reduced)

Following this reaction, the reduced PES then reduces DCIP:

DCIP (oxidized) + PES (reduced) 〉̶ DCIP (reduced) + PES (oxidized)

Applying a positive voltage to the electrode oxidizes the reduced form of DCIP. The oxidation reaction results in an oxidation current that is detected according to the following equation:

DCIP (reduced) - electron 〉̶ DCIP (oxidized)

The results of detection at 300 millivolts are shown in Figure 31, which provides a three dimensional plot of current difference and a two-dimensional schematic representation of the average current difference of the electrode groups having P1, P5, or P6. The darker square indicates a higher average current of the electrodes in that grouping. Figure 31 shows that substantial current is measured over the background current when the target oligonucleotide concentration is 1 nanomolar (P1). The current differences at 10 picomolar (P6) and 50 picomolar (P5) are less than at 1 nanomolar as expected and are approximately equal to each other. The pattern of current differences is repeated when measured at a potential of 500 millivolts as shown in Figure 32. The two-dimensional representations in Figures 31 and 32 do not show the imperfections in the actual photographs.

To obtain a microarray with both HRP and GDH linked oligonucleotides, P1 was used for GDH and P6 was used for HRP. Complementary probes were synthesized *in situ* (standard phosphoramidite chemistry using electrochemical deblocking) on the microarray (CombiMatrix). The microarray was incubated with P6 biotinylated oligonucleotide (0.1 nanomolar, 40 C, 1 hour). The microarray was washed and then incubated with HRP-streptavidin conjugate to attach the HRP to the microarray. The conjugate comprised a proprietary polymer of 80 HRP units conjugated to streptavidin. The streptavidin-HRP complex was purchased from Research Diagnostics, Inc. To make the streptavidin-HRP solution, one microliter of the streptavidin-HRP 80 complex was diluted to 5000 microliters using 2XPBST. The time of exposure was 60 minutes and the solution temperature was 25 degrees Celsius. The resulting linkage was hybridized P6-biotin-streptavidin-HRP. The procedure was then repeated for GDH on the same microarray to obtain hybridized P1-biotin-streptavidin-GDH. HRP detection was performed at standard HRP detection conditions with TMB as a substrate in the presence of hydrogen peroxide. The voltage was -0.2 volts. The solution was a 10 times diluted commercial TMB solution. The temperature was ambient temperature. GDH and HRP were detected sequentially using the different solutions. GDH was detected using the solution described previously in this example.

Figure 33 shows 2-dimensional schematic representations of average current minus average background current. The schematics do not capture the imperfections in the original photographs, which show that there was some shadow at the P1 locations due to contamination from the cover slip. The upper schematic represent GDH detection as the small lighter squares. The lower schematic represents HRP detection as the small lighter squares. During detection of GDH, the electrodes having HRP did not have a current significantly above the background current. Similarly, during detection of HRP, the electrodes having GDH did not have a current significantly above the background current. Thus each enzyme may be detected on the same electrode microarray without interference from the other enzyme. This lack of crosstalk may be exploited to construct a microarray for detection of multiple analytes using more than one type of enzyme.

### Example 14: Redox Enzyme Amplified Detection by Deposition Product

In this example, HRP was used as the enzyme to form an insoluble deposition product on the electrode microarray to detect a binding event. A CombiMatrix CUSTOMARRAY 12K™ electrode microarray was used as the microarray. The insoluble deposition product increased electrical resistance of the electrodes thus providing a measure of the binding event. Four sub-areas were selected for synthesis of four different oligonucleotides for each area, e.g., each area had each of the four oligonucleotides. Each area comprised 16 electrodes (4x4). The chip sub-areas contained the following oligonucleotides: oligo 1, 5'-20-mer H-3'; oligo 2, 5'-20-mer 1-3'; oligo 3, 5'-20-mer J-3'; and oligo 4, 5'-20-mer K-3'. Each oligo was synthesized *in situ* on four electrodes within each sub-area using standard phosphoramidite chemistry and electrochemical deblocking. On the remaining electrodes, numerous random oligonucleotides were synthesized. The microarray was then incubated overnight at 37 degrees Celsius with biotin-containing complementary oligonucleotides to oligos 1-4. Various concentrations of complementary oligonucleotides were used. The microarray was washed extensively and then incubated at ambient temperature with HRP-streptavidin conjugate. The conjugate comprised a proprietary polymer of 80 HRP units conjugated to streptavidin. The streptavidin-HRP complex was purchased from Research Diagnostics, Inc. To make the streptavidin-HRP solution, one microliter of the streptavidin-HRP 80 complex was diluted to 5000 microliters using 2XPBST. The time of exposure was 60 minutes and the solution temperature was 25 degrees Celsius. It was then washed again using 2XPBST, and incubated with DAB-hydrogen peroxide solution for 30 minutes in order to form a deposit layer on the microarray. The concentration of DAB was 1 millimolar. The concentration of hydrogen peroxide was 1 millimolar. The deposit layer was brown and easily could be observed at elevated depositions. Finally, electrochemical detection was performed using different concentrations of K₃[Fe(CN)₆]/K₄[Fe(CN)₆] solution and different applied voltages.

Figure 22 is a black and white representation of a light photomicrograph of the electrode microarray. The insoluble product deposition on microarray sub-areas can be seen on each 4x4 grouping of electrodes. The complementary oligonucleotide concentration was varied from 1 picomolar up to 1 nanomolar. The difference in the deposition of the insoluble material at high and low concentration of captured biotin-containing complementary oligonucleotides can be observed. The current for the electrochemical reduction of K₄[Fe(CN)₆] at the electrodes having complementary oligonucleotides is drastically reduced in contrast the current obtained for sites where random-sequence oligomers were produced (background electrodes). This difference in current can be as high as tens of nanoamperes for high concentration of the redox mediator.

Figure 23 shows two-dimensional images of an electrode microarray displaying electrochemical reduction of 2 millimolar K₃[Fe(CN)₆]/K₄[Fe(CN)₆] at -100 mV on a HRP-DAB-deposit after a 30 minute deposition time. Current decrease is observed for complementary oligonucleotide concentration as low as 10 femtomolar. Above 10 picomolar, saturation of the signal occurs; however, data may be retrieved from these electrodes if the deposition time is reduced.

### Example 15: Redox Enzyme Amplified Detection by Reduced Deposition Product

In this example, HRP was used as the enzyme to deposit an insoluble product on the electrode microarray thereby translating a binding event into a finite amount of redox active material. A CombiMatrix CUSTOMARRAY 12K electrode microarray was used as the microarray. The insoluble deposition product was reduced by the application of a voltage to a counter electrode thus providing a measure of the binding event on the microarray.

The microarray was prepared as a DNA microarray for a complex gene expression assay. Target RNA was hybridized to complimentary probes on the microarray for 18 hours at a temperature of 45 degrees Celsius. The target RNA was biotin labeled. The hybridized sequences were subsequently labeled with poly-HRP streptavidin conjugate. After washing the electrode microarray, it was exposed to tetramethylbenzidine (TMB) with 0.003% hydrogen peroxide for 5 minutes. The insoluble species, oxidized-TMB, deposited in registration with the HRP-labeled target RNA. The TMB reagent was removed and replaced by phospho-citrate buffer plus 200 millimolar NaCl to stop the oxidation reaction. Oxidized TMB is blue in color, and the deposition of this material was readily visible. The buffer solution was redox inactive; therefore, the current observed in the detection step resulted from redox active molecules on the electrode.

The electrode microarray had areas of no deposit, light blue deposit, and dark blue deposit. Figure 24 is a black and white representation of a color image of the electrode microarray displaying the blue deposition product. The dark blue areas indicated a high concentration of bound RNA and subsequent HRP; in contrast, the light blue indicated lower concentrations of both. On the microarray, there were electrodes that had no synthesized probe DNA. These electrodes, as expected, had no deposit. The darker blue regions of the microarray had a high concentration of DNA sequences complementary to targets in solution.

Initially, a small portion of the microarray was electrochemically read at a voltage of-0.1 volts versus an indium-tin oxide (ITO) counter electrode. Figure 25 is a black and white converted image of the electrode microarray showing a depiction of the current at each electrode during the reading of the microarray. The white areas correspond to electrodes having probe DNA and hence, faradaic current owing to the reduction of the deposition product. The black areas represent electrodes not having the deposition product and hence minimal current flow. The bottom row of electrodes had no synthesized DNA; therefore, no HRP was attached and hence no deposition product formed. The current on the electrodes without DNA was approximately 0.2 nanoamperes (mean), and such electrodes are shown in black in Figure 25.

The remaining electrodes in Figure 25 had synthesized probe sequences that had hybridized to RNA in solution. The concentration of the RNA in solution was varied from 0.375 to 12 pM. The current observed at each electrode is a linear combination of faradaic and charging current. In order to discern a difference between electrodes having enzyme and those not having enzyme attached thereto, the charging current must be a negligible fraction of the total current. The specific binding events and subsequent deposition of oxidized TMB yielded a range of currents from 0.5 to 12 nanoamperes, whereas non-specific interactions on negative control probes were less than 0.4 nanoamperes. This technique is capable of detecting specific DNA-RNA interactions in the presence of non-specific interferences.

After acquiring the signal seen in Figure 26, the microarray was read again at -0.1 volts. Since all of the oxidized TMB was reduced during the previous read, the observed current is due only to charging of the electrode. This current, on average, was 0.2-0.3 nanoamperes and is seen in Figure 26. Charging current was observed to be, in some cases, greater than 10 times less the faradaic current on select electrodes. This is advantageous in that much of the observed current in Figure 25 is due to faradaic processes with very little charging interference. The grayscales of Figures 25 and 26 have been independently adjusted to show maximum contrast within the images; however, the black and white representation in the figures looses the grayscale.

To read the entire microarray, a method was implemented as illustrated in Figure 27. In read cycle 0, all electrodes were set to 10 millivolts. This state was concurrent with all electrodes being at open circuit because no low resistance path to ground existed. Each individual electrode was sequentially set to ground (read cycles 1-3) while the remainder of the microarray was held at 10 millivolts. To measure the current, one electrode was set to ground to complete the circuit by initiation of a low resistance pathway to ground through the current measurement electronics. During this time, current was monitored and recorded. Current flow was rectified in one direction, e.g. from the grounded individual electrode to the rest of the microarray due to the applied potential. The current flow reduced material on the grounded electrode and oxidized any material on the remaining electrodes in the microarray. This scheme effectively used the entire microarray as a counter electrode and the grounded electrode as a working electrode. To read the entire microarray, each individual electrode was sequentially switched to ground, the current was recorded then switched back to the applied potential as illustrated in Figure 27. Reading the microarray was done in a sequential manner by reading each electrode individually.

In more detail, each electrode is attached to a multiplexing switch. The switch is initially set to a voltage source (approximately 10 mV). Then, a short time before the current is read, the electrode is switched to true ground for a time that is variable. This time can be 0-10 milliseconds after which the electrode is switched to the input of an amplifier. The input of this amplifier is held close to ground potential within 1 mV, but it isn't truly ground. In most cases the input of the amplifier is adjusted to be as close to ground as possible, but it also has the capability to be adjusted to a voltage not equal to ground. This could be used to null background current for instance. In practice, the voltage is maintained as close to ground as possible. There are different amplifiers that may be used and include: integrating amplifier (this is the style used), transimpedance amplifier, and voltage amplifier.

Figure 28 is a black and white converted image of the electrode microarray showing a depiction of the current at each electrode during the reading of the microarray using the above mention method. The white areas correspond to electrodes having probe DNA and hence faradaic current owing to the reduction of the deposition product. The black areas represent not having the deposition product and hence minimal current flow. The entire CUSTOMARRAY 12K chip was read in 24 seconds with an average background charging current of 0.2 nanoamperes for non-DNA synthesized electrode and electrodes with negative control sequences. The current resulting form various concentrations of labeled RNA targets are seen in Figure 29. A clear increase in current is roughly exponential with a corresponding linear increase in concentration of RNA in solution. This assay illustrates that this method is capable of differentiate of various concentrations of RNA solution without interference from charging current or other currents resulting from non-specific RNA-DNA interactions.

The data indicates that DNA/RNA hybridization can be imaged by electrochemically reducing an immobilized product of the HRP/TMB reaction made in the presence of hydrogen peroxide. This reading method allowed stopping of the enzyme reaction and reading the microarray at a later time. Low over potentials were used so that background current due to charging was minimized, thus improving signal to noise ratio. A separate off-microarray electrode was not necessary for this reading method. All electrodes served as counter electrodes, and all electrodes were available for assay purposes. Additionally, the concentration of target in solution was found to be proportional to the current while there was negligible interference from charging current.

### Example 16: Redox Enzyme Amplified Detection by Conductivity of Solid Deposition Product

In this example, electrochemical detection of oligonucleotide hybridization binding events is performed using HRP as the enzyme and pyrrole and hydrogen peroxide as the substrates. HRP is attached to the target oligonucleotide and catalyzes the polymerization of pyrrole (oxidation) to form polypyrrole, which deposits at the electrodes having the HRP attached thereto. The polypyrrole is a conductive polymer and thus increases the conductivity at the electrodes having polypyrrole deposited thereon. The presence of the polypyrrole is measured subsequent to deposition. As measured at a constant voltage, those electrodes not having the polypyrrole will have a lower current relative to those having the polypyrrole.

In this example, the hybridization of biotinylated amplified RNA (aRNA) transcripts was detected by measuring an increase in conduction at the electrode in which the hybridization occurred. Conduction was measured as an increase in the amount of current passing through the electrode when set to -300mV in a solution of PBS. An increase in conduction at the electrode was achieved by HRP enzymatic deposition of the conducting polymer polypyrrole. After hybridization of the aRNA transcripts, streptavidin-HRP was bound to the biotinylated aRNA. The HRP then oxidized the substrate pyrrole to form polypyrrole, which deposited over the individual electrodes where hybridization occurred. The amount of polypyrrole formed was directly proportional to the amount of HRP, which was directly proportional to the amount biotinylated aRNA that hybridized to the electrode site. Furthermore, the amount of current detected at an electrode set to -300mV was directly proportional to the amount of polypyrrole deposited at the electrode.

Six different 1000 base pairs (bp) DNA sequences with T7 promoters were constructed from Lambda DNA using PCR. The six sequences were designated sp-12, sp-09, sp-08, sp-06, sp-05, and sp-03. Amplified RNA of these transcripts was generated by T7 amplification, and labeling was achieved by incorporation of biotinylated UTP during this amplification. A population of non-specific aRNA was generated from K562 (human CML cell line) mRNA using the Eberwine method for linearly amplifying antisense aRNA from mRNA and labeled with biotin in the same way as the six transcripts. (Phillips J, Eberwine JH. (1996) Antisense RNA Amplification: A Linear Amplification Method for Analyzing the mRNA Population from Single Living Cells. Methods. 10(3):283-288) using licensed reagents. All aRNA was fragmented to between 50 and 200 bp lengths using Magnesium 2+ induced metal hydrolysis.

The six transcripts, sp-12, sp-09, sp-08, sp-06, sp-05, and sp-03, along with 5 micrograms of fragmented K562 aRNA were hybridized to a CombiMatrix CUSTOMARRAY™ 12k DNA electrode microarray for 16 hours at 45°C in 6xSSPE solution with 0.1% TWEEN™ and 25% formamide. The microarray contained probes for K562 specific genes as well as three different probes that were specific for each of the six transcripts. All probes ranged from 35 to 40 bp in length. The six transcripts, sp-12, sp-09, sp-08, sp-06, sp-05, and sp-03, were hybridized at the following concentrations, respectively: 3000, 1000, 300, 100, 10, and 1 picomolar.

After hybridization, the microarray was washed and incubated with streptavidin-HRP for 15 minutes. The microarray was washed and incubated for one hour in 50 millimolar phosphate-citrate buffer at pH 5 (Sigma #P-4809) with 0.1% pyrrole and 0.03% hydrogen peroxide. The microarray was washed three times with 1x PBS. The microarray was left in 1x PBS for electrochemically detection of the polypyrrole. To measure current, the electrode to be read was set to ground while an off-microarray electrode was set to 300 millivolts. The current measurement was taken as the amount of current (I) passing to ground.

The amount of current passing is directly proportional to the amount of polypyrrole present. In Figure 35, a two dimensional depiction is provided of the amount of current passing at different electrodes. The figure is a grayscale image of the current passing in each electrode, which is designated as a square block. A higher current corresponds to a lighter image in a block. This figure shows the three probes for each of the six transcripts hybridized (in columns per transcript) as well as the concentration in solution of the hybridized transcript (bottom row). There are also nine negative control probes that have no hybridizing species in solution. In Figure 36, the numeric value of the current at the electrodes for each hybridized transcript is provided. The value is the average of the three different probes for each transcript concentration. In Figure 37, the amount of increase relative to the average background is shown as calculated from the nine zero concentration probes. The figure shows that current increased 1 times at the lowest concentration to 4.5 times at the highest concentration.

## Claims

1. A process to detect binding events on an electrode microarray using enzymes comprising:
(a) providing a microarray having a plurality of electrodes and a plurality of capture complexes of at least two different types and attached at sites corresponding to the electrodes, wherein a first capture complex type has affinity for a first analyte type and subsequent capture complex types have affinity for subsequent analyte types, wherein the electrodes are electronically addressable, and wherein an addressable electrode is one where the electrode can be electronically controlled to create a current or voltage at the electrode;
(b) administering a plurality of analytes of at least one type to the capture complexes to form bound complexes of types corresponding to each type of the analytes captured by the capture complexes;
(c) attaching enzymes to the bound complexes to form reporter complexes, wherein the reporter complexes are of a type corresponding to each type of the analytes captured by the capture complexes;
(d) sequentially contacting a plurality of substrate solutions to the microarray and measuring for the presence or absence of an enzymatic product at the sites using an electrical signal, wherein each substrate solution corresponds to the reporter complex type, wherein the presence of the electrical signal is a measure of the binding event, and
wherein a low initial potential slightly positive to ground, preferably approximately 10 millivolt absolute, is applied to the electrodes while maintaining an open circuit to the electrodes; and wherein an electrical response of each electrode is measured by sequentially setting each electrode to ground, measuring the current flow from each electrode towards the remaining part of the electrode microarray used as a counter electrode, and returning each electrode to the initial potential at open circuit.

2. The process of claim 1, wherein the enzyme product in step d) is a solid enzyme product that deposits on the sites having enzymes, and wherein a measurement solution and an electrical signal are used for the presence or absence of the solid enzyme product at the sites.

3. The process of claim 1 or 2, wherein the capture complexes are comprised of a plurality of probe oligonucleotides, the analytes are comprised of a plurality of target oligonucleotides, the bound complexes are formed by hybridization of the target oligonucleotides to the probe oligonucleotides, and the enzymes are bound to the target oligonucleotides by an attaching method, wherein the attaching method comprises attaching the enzymes through molecular groups selected from the group consisting of (a) an antibody, anti-antibody, and anti-idiotype antibody combination, (b) a biotin and streptavidin or avidin combination, and (c) an oligonucleotide and complementary oligonucleotide combination, and combinations thereof.

4. The process of claim 1 or 2, wherein the capture complexes are comprised of probe oligonucleotides hybridized to target oligonucleotides having antibody tags, the bound complexes are formed by capture of the analytes by the antibody tags, and the enzymes are bound to the analytes by an attaching method, wherein the attaching method comprises a reporter antibody attached to the analyte and the enzyme attached to the reporter antibody through molecular groups selected from the group consisting of (a) an antibody, anti-antibody, and anti-idiotype antibody combination, (b) a biotin and streptavidin or avidin combination, and (c) an oligonucleotide and complementary oligonucleotide combination, and combinations thereof.

5. The process of claim 3 or 4, wherein the probe oligonucleotides are synthesized by *in situ* electrochemical synthesis.

6. The process of claim 1 or 2, wherein the capture complexes are made by a method selected from the group consisting of *in situ* electrochemical synthesis, spotting, ink-jet printing, electric field deposition, and *in situ* photolithography synthesis.

7. The process of claim 1 or 2, wherein the capture complexes are comprised of molecules selected from the group consisting of oligonucleotides, polypeptides, antibodies, glycosylated polypeptides, polysaccharides, peptide nucleic acids, and mixed molecules having monomers from a plurality of the foregoing molecules.

8. The process of claim 1 or 2, wherein the analytes are comprised of molecules selected from the group consisting of antigens, haptens, viruses, bacteria, cells, proteins, polysugars, biological polymer molecules, lipids, glycoproteins (alpha-1-acid glycoprotein,) ricin, M13 phage, Bacillus globigii (BG) spores, fluorescein, rabbit IgG, goat IgG, DNA, RNA, single-stranded DNA, ribosomal RNA, mitochondrial DNA, cellular receptors, glycosylated membrane-bound proteins, non-glycosylated membrane-bound proteins, polypeptides, glycosylated polypeptides, antibodies, cellular antigenic determinants, organic molecules, metal ions, salt anions and cations, and organometallics, and combinations thereof.

9. The process of claim 1 or 2, wherein the enzymes are selected from the group consisting of horseradish peroxidase, laccase, beta-galactosidase, glucose oxidase, alkaline phosphatase, glucose-6-phosphate dehydrogenase, catalase, lactate oxidase, and peroxidase, and combinations thereof.

10. The process of claim 1 or 2, wherein the substrate solutions are comprised of an aqueous solution having a buffer, a salt, and an enzymatic substrate solution, wherein the enzymatic substrate solution has substrates that are reactive with the enzyme.

11. The process of claim 2, wherein the substrate solutions are comprised of an aqueous solution having a buffer, a salt, and an enzymatic substrate solution, wherein the enzymatic substrate solution has substrates that are reactive with the enzyme, wherein the enzyme is horseradish peroxidase and the substrate is hydrogen peroxide and a chemical selected from the group consisting of oxidizeable aromatics, ferrocene derivatives, and oxidizeable inorganic compounds, pyrrole, and combinations thereof.

12. The process of claim 1 or 2, wherein the electrical signal comprises a difference response, wherein the difference response is a measure of the difference between a product response and a base response, wherein the product response is measured on the sites having enzyme and the base response is measured on sites not having enzyme, wherein the product response and the base response are selected from the group consisting of current, voltage, and resistance, wherein the difference response is a measure of binding of the analytes to the capture complexes.

13. The process of claim 1, wherein the enzymatic product comprises a molecule that is electrochemically reducible or oxidizeable.

14. The process of claim 2, wherein the solid enzyme product is:
A) non-reactive and the measurement solution is an aqueous solution having an electron mediator, preferably the electron mediator being a mixture of potassium ferrous cyanide and potassium ferric cyanide having a mixture proportion of approximately 10:90 to 90:10 and a concentration of approximately 0.1 to 1000 millimolar, or
B) reducible or oxidizeable and the measurement solution is an aqueous solution having a buffer and a salt, preferably the buffer being a phosphate-citrate buffer and the salt being sodium chloride, or
C) a conductive material, preferably being polypyrrole.

15. The process of any one of claims 1 to 14, the measurement time is approximately 0.1 milliseconds to 1 second.

## Patentansprüche

1. Verfahren zum Erfassen von Bindungsvorgängen an einem Elektroden-Microarray unter Verwendung von Enzymen, umfassend:
(a) Bereitstellen eines Microarrays mit einer Vielzahl an Elektroden und einer Vielzahl an Einfangkomplexen (capture complexes) von mindestens zwei unterschiedlichen Typen und an Stellen befestigt, die zu den Elektroden korrespondieren, wobei ein erster Einfangkomplextyp eine Affinität für einen ersten Analytentypen hat und nachfolgende Einfangkomplextypen Affinitäten für nachfolgende Analytentypen haben, wobei die Elektroden elektronisch ansprechbar sind und wobei eine ansprechbare Elektrode eine ist, wo die Elektrode elektronisch kontrolliert werden kann, um einen Strom oder eine Spannung an der Elektrode zu erzeugen;
(b) Verabreichen einer Vielzahl an Analyten von mindestens einem Typ an die Einfangkomplexe, um gebundene Komplexe zu bilden von Typen, die jedem Typ der Analyten, die durch die Einfangkomplexe eingefangen werden, entsprechen;
(c) Befestigen von Enzymen an die gebundenen Komplexe, um Reporterkomplexe zu bilden, wobei die Reporterkomplexe von einem Typ sind, die jedem Typ der Analyten, die durch die Einfangkomplexe eingefangen werden, entspricht;
(d) nacheinander eine Vielzahl an Substratlösungen in Kontakt mit dem Microarray bringen und messen in Bezug auf die Gegenwart oder Abwesenheit eines enzymatischen Produktes an den Stellen unter Verwendung eines elektrischen Signals, wobei jede Substratlösung dem Reporterkomplextyp entspricht, wobei die Gegenwart des elektrischen Signals eine Messung des Bindungsvorgangs ist, und
wobei ein niedriges Anfangspotential, dass leicht positiv gegenüber dem Bezugspotential ist, bevorzugt ungefähr 10 Millivolt absolut, an den Elektroden angelegt wird, während ein offener Stromkreis zu den Elektroden gehalten wird; und wobei eine elektrische Antwort jeder Elektrode gemessen wird durch aufeinanderfolgendes Erden jeder Elektrode, Messen des Stromflusses von jeder Elektrode zum verbleibenden Teil des Elektroden-Microarrays, welcher als Gegenelektrode verwendet wird, und Zurückführen jeder Elektrode auf das Anfangspotential bei offenem Stromkreis.

2. Das Verfahren gemäß Anspruch 1, wobei das Enzymprodukt in Schritt (d) ein festes Enzymprodukt ist, welches sich an den Stellen mit Enzymen absetzt und wobei eine Messlösung und ein elektrisches Signal verwendet werden für die Gegenwart oder Abwesenheit des festen Enzymproduktes an den Stellen.

3. Das Verfahren gemäß Anspruch 1 oder 2, wobei die Einfangkomplexe eine Vielzahl an Sonden-Oligonukleotiden umfassen, die Analyten eine Vielzahl an Ziel-Oligonukleotiden umfassen, die gebundenen Komplexe durch Hybridisierung der Ziel-Oligonukleotide an die Sonden-Oligonukleotide gebildet werden und die Enzyme an die Ziel-Oligonukleotide durch eine Befestigungsmethode gebunden werden, wobei die Befestigungsmethode das Befestigen der Enzyme durch molekulare Gruppen, ausgewählt aus der Gruppe bestehend aus (a) einer Antikörper-, Anti-Antikörper- und Anti-Idiotyp-Antikörper-Kombination, (b) einer Biotin- und Streptavidin- oder Avidin-Kombination und (c) einer Oligonukleotid- und komplementären Oligonukleotid-Kombination und Kombinationen daraus, umfasst.

4. Das Verfahren gemäß Anspruch 1 oder 2, wobei die Einfangkomplexe Sonden- Oligonukleotide umfassen, die an Ziel-Oligonukleotide mit Antikörper-Markierungen hybridisiert sind, die gebundenen Komplexe durch Einfangen der Analyten durch die Antikörper Markierungen gebildet werden, und die Enzyme an die Analyten durch eine Befestigungsmethode gebunden sind, wobei die Befestigungsmethode einen Reporter-Antikörper umfasst, der an dem Analyten befestigt ist und das Enzym, das an dem Reporter-Antikörper durch molekulare Gruppen befestigt ist, die ausgewählt sind aus der Gruppe bestehend aus (a) einer Antikörper-, Anti-Antikörper- und Anti-Idiotyp-Antikörper-Kombination, (b) einer Biotin- und Streptavidin- oder Avidin-Kombination und (c) einer Oligonukleotid- und komplementären Oligonukleotid-Kombination und Kombinationen daraus.

5. Das Verfahren gemäß Anspruch 3 oder 4, wobei die Sonden-Oligonukleotide durch *in situ* elektrochemische Synthese synthetisiert werden.

6. Das Verfahren gemäß Anspruch 1 oder 2, wobei die Einfangkomplexe durch ein Verfahren ausgewählt aus der Gruppe bestehend aus *in situ* elektrochemischer Synthese, Spotting, Tintenstrahldrucken, Abscheidung im elektrischen Feld und *in situ* Synthese durch Photolithographie hergestellt werden.

7. Das Verfahren gemäß Anspruch 1 oder 2, wobei die Einfangkomplexe Moleküle umfassen, die ausgewählt sind aus der Gruppe bestehend aus Oligonukleotiden, Polypeptiden, Antikörpern, glykosylierten Polypeptiden, Polysacchariden, Peptid-Nukleinsäuren und gemischten Molekülen mit Monomeren aus einer Vielzahl der vorangegangenen Molekülen.

8. Das Verfahren gemäß Anspruch 1 oder 2, wobei die Analyten Moleküle umfassen, die ausgewählt sind aus der Gruppe bestehend aus Antigenen, Haptenen, Viren, Bakterien, Zellen, Proteinen, Mehrfachzuckern, biologischen Polymermolekülen, Lipiden, Glykoproteinen (saures Alpha1-Glykoprotein (Orosomucoid)), Rizin, M13-Phage, Sporen von Bacillus Globigii (BG), Fluorescein, Kaninchen-IgG, Ziegen-IgG, DNA, RNA, einsträngiger DNA, ribosomaler RNA, mitochondrialer DNA, zellulären Rezeptoren, glykosylierten membrangebundenen Proteinen, nichtglykosylierten membrangebundenen Proteinen, Polypeptiden, glykosylierten Polypeptiden, Antikörpern, zellulären Epitopen, organischen Molekülen, Metallionen, Salzanionen und -kationen und metallorganischen Verbindungen und Kombinationen daraus.

9. Das Verfahren gemäß Anspruch 1 oder 2, wobei die Enzyme ausgewählt sind aus der Gruppe bestehend aus Meerrettichperoxidase, Laccase, β-Galactosidase, Glukoseoxidase, alkalische Phosphatase, Glukose-6-phosphat-Dehydrogenase, Katalase, Lactatoxidase und Peroxidase und Kombinationen daraus.

10. Das Verfahren gemäß Anspruch 1 oder 2, wobei die Substratlösungen eine wässrige Lösung mit einem Puffer, einem Salz und einer enzymatischen Substratlösung umfassen, wobei die enzymatische Substratlösung Substrate aufweist, die mit dem Enzym reagieren.

11. Das Verfahren gemäß Anspruch 2, wobei die Substratlösungen eine wässrige Lösung mit einem Puffer, einem Salz und einer enzymatischen Substratlösung umfassen, wobei die enzymatische Substratlösung Substrate aufweist, welche mit dem Enzym reagieren, wobei das Enzym Meerrettichperoxidase ist und das Substrat Wasserstoffperoxid und eine Chemikalie, ausgewählt aus der Gruppe bestehend aus oxidierbaren aromatischen Verbindungen, FerrocenDerivaten und oxidierbaren anorganischen Verbindungen, Pyrrol und Kombinationen daraus ist.

12. Das Verfahren gemäß Anspruch 1 oder 2, wobei das elektrische Signal eine differenzierte Antwort umfasst, wobei die differenzierte Antwort die Messung der Differenz zwischen einer Produktantwort und einer Basisantwort ist, wobei die Produktantwort an den Stellen mit Enzym gemessen wird und die Basisantwort an den Stellen ohne Enzym gemessen wird, wobei die Produktantwort und die Basisantwort ausgewählt sind aus der Gruppe bestehend aus Strom, Spannung und Widerstand, wobei die differenzierte Antwort eine Messung der Bindung der Analyten an die Einfangkomplexe ist.

13. Das Verfahren gemäß Anspruch 1, wobei das enzymatische Produkt ein Molekül umfasst, welches elektrochemisch reduzierbar oder oxidierbar ist.

14. Das Verfahren gemäß Anspruch 2, wobei das feste Enzymprodukt ist:
(A) nicht reaktiv und die Messlösung ist eine wässrige Lösung mit einem Elektronenvermittler, der Elektronenvermittler ist bevorzugt eine Mischung aus Kaliumhexacyanoferrat(II) und Kaliumhexacyanoferrat(III) mit einem Mischungsverhältnis von ungefähr 10 : 90 bis 90 : 10 und einer Konzentration von ungefähr 0,1 bis 1.000 Millimolar, oder
(B) reduzierbar oder oxidierbar und die Messlösung ist eine wässrige Lösung mit einem Puffer und einem Salz, der Puffer ist bevorzugt ein Phosphat-Citrat-Puffer und das Salz ist Natriumchlorid oder
(C) ein leitendes Material, bevorzugt ist Polypyrrol.

15. Das Verfahren gemäß einem der Ansprüche 1 bis 14, die Messzeit ist ungefähr 0,1 Millisekunden bis 1 Sekunde.

## Revendications

1. Procédé de détection d'événements de liaison sur un micro-réseau d'électrodes utilisant des enzymes, ledit procédé comprenant les étapes consistant à :
(a) fournir un micro-réseau ayant une pluralité d'électrodes et une pluralité de complexes de capture d'au moins deux types différents et fixés à des sites correspondant aux électrodes, un premier type de complexe de capture ayant une affinité pour un premier type d'analyte et d'autres types de complexes de capture ayant une affinité pour d'autres types d'analytes, les électrodes étant électroniquement adressables, et une électrode adressable étant une électrode qui peut être commandée électroniquement pour créer un courant ou une tension au niveau de l'électrode ;
(b) administrer une pluralité d'analytes d'au moins un type à des complexes de capture afin de former des complexes liés de types correspondant à chaque type d'analytes capturés par les complexes de capture ;
(c) fixer des enzymes aux complexes liés afin de former des complexes rapporteurs, les complexes rapporteurs étant d'un type correspondant à chaque type d'analytes capturés par les complexes de capture ;
(d) mettre séquentiellement une pluralité de solutions de substrats en contact avec le micro-réseau et effectuer des mesures pour déterminer la présence ou l'absence d'un produit enzymatique au niveau des sites en utilisant un signal électrique, chaque solution de substrat correspondant au type de complexe rapporteur, la présence du signal électrique étant une mesure de l'événement de liaison, et
dans lequel un potentiel initial faible légèrement positif par rapport à la terre, de préférence d'environ 10 millivolts absolus, est appliqué aux électrodes tout en maintenant les électrodes en circuit ouvert ; et dans lequel une réponse électrique de chaque électrode est mesurée par mise à la terre de chaque électrode de manière séquentielle, par mesure du flux de courant depuis chaque électrode en direction de la partie restante du micro-réseau d'électrodes utilisée comme contre-électrode, et par retour de chaque électrode au potentiel initial en circuit ouvert.

2. Procédé selon la revendication 1, dans lequel le produit enzymatique à l'étape d) est un produit enzymatique solide qui se dépose au niveau des sites où il y a des enzymes, et dans lequel une solution de mesure et un signal électrique sont utilisés pour détecter la présence ou l'absence du produit enzymatique solide au niveau des sites.

3. Procédé selon la revendication 1 ou 2, dans lequel les complexes de capture sont constitués d'une pluralité de sondes d'oligonucléotides, les analytes sont constitués d'une pluralité d'oligonucléotides cibles, les complexes liés sont formés par hybridation des oligonucléotides cibles aux sondes d'oligonucléotides, et les enzymes sont liées aux oligonucléotides cibles par un procédé de fixation, le procédé de fixation comprenant la fixation des enzymes au moyen de groupes moléculaires choisis dans le groupe constitué par (a) un anticorps, un anti-anticorps, et la combinaison d'anticorps anti-idiotypiques, (b) une combinaison de biotine et de streptavidine ou d'avidine, et (c) une combinaison d'un oligonucléotide et d'un oligonucléotide complémentaire, et des combinaisons de ceux-ci.

4. Procédé selon la revendication 1 ou 2, dans lequel les complexes de capture sont constitués de sondes d'oligonucléotides hybridées à des oligonucléotides cibles comportant des marqueurs d'anticorps, les complexes liés sont formés par capture des analytes par les marqueurs d'anticorps, et les enzymes sont liées aux analytes par un procédé de fixation, le procédé de fixation comprenant un anticorps rapporteur fixé à l'analyte et l'enzyme fixée à l'anticorps rapporteur à l'aide de groupes moléculaires choisis dans le groupe constitué par (a) un anticorps, un anti-anticorps et une combinaison d'anticorps anti-idiotypiques, (b) une combinaison de biotine et de streptavidine ou d'avidine, et (c) une combinaison d'un oligonucléotide et d'un oligonucléotide complémentaire, et des combinaisons de ceux-ci.

5. Procédé selon la revendication 3 ou 4, dans lequel les sondes d'oligonucléotides sont synthétisées par synthèse électrochimique *in situ.*

6. Procédé selon la revendication 1 ou 2, dans lequel les complexes de capture sont réalisés par un procédé choisi dans le groupe comprenant la synthèse électrochimique *in situ,* la formation de plots, l'impression par jet d'encre, le dépôt par champ électrique, et la synthèse par photolithographie *in situ.*

7. Procédé selon la revendication 1 ou 2, dans lequel les complexes de capture comprennent des molécules choisies dans le groupe constitué par les oligonucléotides, les polypeptides les anticorps, les polypeptides glycosylés, les polysaccharides, les acides nucléiques peptidiques, et des molécules mixtes comportant des monomères provenant de plusieurs des molécules précédentes.

8. Procédé selon la revendication 1 ou 2, dans lequel les analytes comprennent des molécules choisies dans le groupe constitué par les antigènes, les haptènes, les virus, les bactéries, les cellules, les protéines, les polysaccharides, les molécules de polymères biologiques, les lipides, les glycoprotéines (alpha-1-acide glycoprotéine), la ricine, le phage M13, les spores de Bacillus globigii (BG), la fluorescéine, l'IgG du lapin, l'IgG de la chèvre, l'ADN, l'ARN, l'ADN monobrin, l'ARN ribosomique, l'ADN mitochondrial, les récepteurs cellulaires, les protéines membranaires glycosylées, les protéines membranaires non-glycosylées, les polypeptides, les polypeptides glycosylés, les anticorps, les déterminants antigéniques cellulaires, les molécules organiques, les ions métalliques, les anions et cations de sels, et les organométalliques, et des combinaisons de ceux-ci.

9. Procédé selon la revendication 1 ou 2, dans lequel les enzymes sont choisies dans le groupe constitué par la peroxydase de raifort, la laccase, la beta-galactosidase, la glucose oxydase, la phosphatase alcaline, la glucose-6-phosphate déshydrogénase, la catalase, la lactate oxydase, et la peroxydase, et des combinaisons de celles-ci.

10. Procédé selon la revendication 1 ou 2, dans lequel les solutions de substrats comprennent une solution aqueuse comportant un tampon, un sel, une solution de substrats enzymatiques, la solution de substrats enzymatiques comportant des substrats qui sont réactifs vis-à-vis de l'enzyme.

11. Procédé selon la revendication 2, dans lequel les solutions de substrats comprennent une solution aqueuse comportant un tampon, un sel, et une solution de substrats enzymatiques, la solution de substrats enzymatiques comportant des substrats qui sont réactifs vis-à-vis de l'enzyme, l'enzyme étant la peroxydase de raifort et le substrat étant le peroxyde d'hydrogène et un produit chimique choisi dans le groupe constitué par les composés aromatiques oxydables, les dérivés du ferrocène et des composés inorganiques oxydables, les pyrroles et des combinaisons de ceux-ci.

12. Procédé selon la revendication 1 ou 2, dans lequel le signal électrique comprend une réponse différentielle, dans lequel la réponse différentielle est une mesure de la différence entre une réponse de produit et une réponse de base, dans lequel la réponse de produit est mesurée au niveau des sites comportant l'enzyme et la réponse de base est mesurée au niveau des sites ne comportant pas l'enzyme, dans lequel la réponse de produit et la réponse de base sont choisies dans le groupe constitué par l'intensité, la tension, et la résistance, la réponse différentielle étant une mesure de la liaison des analytes aux complexes de capture.

13. Procédé selon la revendication 1, dans lequel le produit enzymatique comprend une molécule qui est réductible ou oxydable par voie électrochimique.

14. Procédé selon la revendication 2, dans lequel le produit enzymatique solide est :
A) non réactif et la solution de mesure est une solution aqueuse ayant un médiateur d'électrons, le médiateur d'électrons étant de préférence un mélange de cyanure de potassium ferreux et de cyanure de potassium ferrique dont la proportion de mélange est d'environ 10:90 à 90:10 et dont la concentration est d'environ 0,1 à 1000 millimolaires, ou
B) réductible ou oxydable et la solution de mesure est une solution aqueuse comportant un tampon et un sel, le tampon étant de préférence un tampon phosphate-citrate et le sel étant du chlorure de sodium, ou
C) un matériau conducteur, de préférence du polypyrrole.

15. Procédé selon l'une quelconque des revendications 1 à 14, dans lequel le temps de mesure est d'environ 0,1 millisecondes à 1 seconde.
